# EUROPEAN PATENT SPECIFICATION

(11) **EP 1 294 872 B1**
(45) Date of publication and mention of the grant of the patent: **31.12.2008**
(21) Application number: 01926743.4
(22) Date of filing: 06.04.2001
(51) Int. Cl.: C12N 15/12, C07K 14/47, C07K 16/18, C12Q 1/68, G01N 33/53, A61K 38/17, A61K 39/395, A61K 48/00

(54) **HUMAN TRP-LIKE CALCIUM CHANNEL PROTEIN-2 (TLCC-2)**
HUMANES TRP-ÄHNLICHES KALZIUMKANALPROTEIN-2 TLCC-2
PROTEINE-2 (TLCC-2) DE CANAL CALCIQUE DU TYPE TRP HUMAIN

(30) Priority: 07.04.2000 US 544797
(43) Date of publication of application: 26.03.2003
(73) Proprietor: MILLENNIUM PHARMACEUTICALS, INC., Cambridge, MA 02139 (US)
(72) Inventor: CURTIS, Rory, A., J., Framingham, MA 01702 (US); SILOS-SANTIAGO, Inmaculada, Cambridge, MA 02138 (US)
(74) Representative: Taylor, Kate Laura
(86) International application number: PCT/US2001/011442
(87) International publication number: WO 2001/077331

(56) References cited:
- WO-A-00/17222
- WO-A-01/12662
- DATABASE EMBL, HEIDELBERG, FRG [Online] 11 December 1998 (1998-12-11) NCI-CGAP: "tb24a12.x1 NCI_CGAP_Kid12 Homo sapiens cDNA clone IMAGE: 2055262 3' similar to WP: R13A5.1 CE01370, mRNA sequence" Database accession no. AI307240 XP002176007 cited in the application
- DATABASE EMBL, HEIDELBERG, FRG [Online] 12 July 1999 (1999-07-12) HILLIER, L. ET AL.: "au44h03.x1 Schneider fetal brain 00004 Homo sapiens cDNA clone IMAGE: 2517653 3' similar to WP: R13A5.1 CE01370, mRNA sequence" Database accession no. AI816064 XP002176008 cited in the application -& DATABASE EMBL, HEIDELBERG, FRG [Online] 12 July 1999 (1999-07-12) HILLIER, L. ET AL.: "au44h03.y1 Schneider fetal brain 00004 Homo sapiens cDNA clone IMAGE: 2517653 5' similar to WP: R13A5.1 CE01370, mRNA sequence" Database accession no. AI815981 XP002176009
- SUN, M. ET AL.: "Mucolipidosis type IV is caused by mutations in a gene encoding a novel transient receptor potential channel" HUMAN MOLECULAR GENETICS, vol. 9, no. 17, 12 October 2000 (2000-10-12), pages 2471-2478, XP002176006 -& DATABASE EMBL, HEIDELBERG, FRG [Online] 1 March 2001 (2001-03-01) SUN, M. ET AL.: "CDNA: FLJ22449 FIS, CLONE HRC09609 (MUCOLIPIN) (MUCOLIPIDOSIS TYPE IV PROTEIN) (MUCOLIPIN 1)" retrieved from HOMO SAPIENS Database accession no. Q9GZU1 XP002176010

## Description

### Related Applications

The present application is a continuation-in-part of U.S. Patent Application Serial No. 09/544,797 entitled "54420, A NOVEL HUMAN CALCIUM CHANNEL", filed April 7, 2000.

### Background of the Invention

Calcium signaling has been implicated in the regulation of a variety of cellular responses, such as growth and differentiation. There are two general methods by which intracellular concentrations of calcium ions may be increased: calcium ions may be brought into the cell from the extracellular milieu through the use of specific channels in the cellular membrane, or calcium ions may be freed from intracellular stores, again being transported by specific membrane channels in the storage organelle. In the situation in which the intracellular stores of calcium have been depleted, a specific type of calcium channel, termed a 'capacitative calcium channel' or a 'store-operated calcium channel' (SOC), is activated in the plasma membrane to import calcium ions from the extracellular environment to the cytosol (for review, see Putney and McKay (1999) BioEssays21:38-46).

Members of the capacitative calcium channel family include the calcium release-activated calcium current (CRAC) (Hoth and Penner (1992) Nature355: 353-355), calcium release-activated nonselective cation current (CRANC) (Krause et al. (1996) J. Biol. Chem.271: 32523-32528), and the transient receptor potential (TRP) proteins. There is no single electrophysological profile characteristic of the family; rather, a wide array of single channel conductances, cation selectivity, and current properties have been observed for different specific channels. Further, in several instances it has been demonstrated that homo- or heteropolymerization of the channel molecule may occur, further changing the channel properties from that of the single molecule. In general, though, these channels function similarly, in that they are calcium ion-permeable cation channels which become activated upon stimulation of phospholipase C_{β} by a G protein-coupled receptor. Depletion of intracellular calcium stores activate these channels by a mechanism which is yet undefined, but which has been demonstrated to involve a diffusible factor using studies in which calcium stores were artificially depleted (*e*.*g*., by the introduction of chelators into the cell, by activating phospholipase C_{γ}, or by inhibiting those enzymes responsible for pumping calcium ions into the stores or those enzymes responsible for maintaining resting intracellular calcium ion concentrations) (Putney, J.W., (1986) Cell Calcium 7: 1-12; Putney, J.W. (1990) Cell Calcium 11:611-624).

The TRP channel family is one of the best characterized members of the capacitative calcium channel group. These channels include transient receptor potential protein and homologues thereof (to date, seven homologs and splice variants have been identified in a variety of organisms), the vanilloid receptors (also known as the capsaicin receptors), stretch-inhibitable non-selective cation channel (SIC), olfactory, mechanosensitive channel, insulin-like growth factor I-regulated calcium channel, and vitamin D-responsive apical, epithelial calcium channel (ECaC), melastatin, and the polycystic kidney disease protein family (see, *e*.*g*., Montell and Rubin (1989) Neuron 2:1313-1323; Caterina et al. (1997) Nature 389: 816-824; Suzuki et al. (1999) J. Biol. Chem. 274: 6330-6335; Kiselyov et al. (1998) Nature 396: 478-482; Hoenderop et al. (1999) J. Biol. Chem. 274: 8375-8378; and Chen et al. (1999) Nature 401(6751): 383-6). Each of these molecules is 700 or more amino acids in length (TRP and TRP homologs have 1300 or more amino acid residues), and shares certain conserved structural features. Predominant among these structural features are six transmembrane domains, with an additional hydrophobic loop present between the fifth and sixth transmembrane domains. It is believed that this loop is integral to the activity of the pore of the channel formed upon membrane insertion (Hardie and Minke (1993) Trends Neurosci 16:371-376). TRP channel proteins also include one or more ankyrin domains and frequently display a proline-rich region at the N-terminus. Although found in disparate tissues and organisms, members of the TRP channel protein family all serve to transduce signals by means of calcium entry into cells, particularly pain signals (see, *e*.*g*., McClesky and Gold (1999) Annu. Rev. Physiol. 61: 835-856), light signals (Hardie and Minke, *supra*), or olfactory signals (Colbert et al. (1997) J. Neurosci 17(21): 8259-8269). Thus, this family of molecules may play important roles in sensory signal transduction in general.

Vanilloid receptors (VRs) are non-selective cation channels that are structurally related to members of the TRP family of ion channels. These receptors have been proposed to mediate the entry of extracellular calcium into cells in response to the depletion of intracellular calcium stores. VRs are expressed in nociceptive neurons, as well as other cells types, and are activated by a variety of stimuli including noxious heat and protons. Capsaicin, which is a well-known agonist of VRs, induces pain behavior in humans and rodents. VR-1, a vanilloid receptor, was identified in rat sensory ganglia (Caterina M. J. et al., (1997) Nature 389:816-824). It has been shown that VR-1 knockout mice are impaired in their detection of painful heat, exhibit no vanilloid-evoked pain behavior, and show little thermal hypersensitivity after inflammation (Szallasi and Blumberg (1999) Pharmacol. Rev. 51:159-211; Tominaga, et al. (1998) Neuron 21:531; Caterina et al. (2000) Science 288:306).

### Summary of the Invention

The present disclosure is based, at least in part, on the discovery of novel transient receptor potential (TRP) (*e*.*g*., the calcium channel and/or vanilloid receptor) family members, referred to herein as TRP-like calcium channel or TLCC-2 nucleic acid and protein molecules. The TLCC-2 molecules of the present invention are useful as targets for developing modulating agents to regulate a variety of cellular processes, including membrane excitability, neurite outgrowth and synaptogenesis, signal transduction, cell proliferation, growth, differentiation, and migration, and nociception In one aspect the invention provides a method for identifying a compound which binds to a polypeptide or modulates the ability of a polypeptide to transport calcium across a membrane, wherein the polypeptide is selected from the group consisting of:
(i) a fragment of a polypeptide comprising the amino acid sequence of SEQ ID NO:2, wherein the fragment comprises at least 250 contiguous amino acids of SEQ ID NO:2;
(ii) a polypeptide which is encoded by a nucleic acid molecule comprising a nucleotide sequence which is at least 99% identical to a nucleic acid comprising the nucleotide sequence of SEQ ID NO:1 or SEQ ID NO:3;
(iii) a polypeptide comprising an amino acid sequence which is at least 90% identical to the amino acid sequence of SEQ ID NO:2; and
(iv) a polypeptide comprising the amino acid sequence of SEQ ID NO:2, comprising:
   (a) contacting the polypeptide, or a cell expressing the polypeptide with a test compound; and
   (b) determining whether the compound binds to the polypeptide or modulates the ability of the polypeptide to transport calcium across a membrane, thereby identifying a compound which binds to the polypeptide or modulates the ability of the polypeptide to transport calcium across a membrane.

In a further aspect the invention provides a method of identifying a nucleic acid molecule associated with a pain disorder comprising:
(a) contacting a sample comprising nucleic acid molecules with a hybridization probe comprising at least 25 contiguous nucleotides of SEQ ID NO:1; and
(b) detecting the presence of a nucleic acid molecule in said sample that hybridizes to said probe, thereby identifying a nucleic acid molecule associated with a pain disorder.

In a further aspect the invention provides method of identifying a nucleic acid associated with a pain disorder comprising:
(a) contacting a sample comprising nucleic acid molecules with a first and a second amplification primer, said first primer comprising at least 25 contiguous nucleotides of SEQ ID NO:1 and said second primer comprising at least 25 contiguous nucleotides from the complement of SEQ ID NO:1;
(b) incubating said sample under conditions that allow nucleic acid amplification; and
(c) detecting the presence of a nucleic acid molecule in said sample that is amplified, thereby identifying a nucleic acid molecule associated with a pain disorder.

In a further aspect the invention provides method of identifying a polypeptide associated with a pain disorder comprising:
(a) contacting a sample comprising polypeptides with a an antibody that selectively binds to a polypeptide of SEQ ID NO:2; and
(b) detecting the presence of a polypeptide in said sample that binds to said antibody, thereby identifying a polypeptide associated with a pain disorder. Preferably said antibody is detectably labeled.

In a further aspect the invention provides a method for identifying a compound capable of treating a pain disorder comprising assaying the ability of the compound or agent to modulate the expression of a nucleic acid molecule selected from from the group consisting of:
(a) a nucleic acid molecule comprising the nucleotide sequence set forth in SEQ ID NO:1;
(b) a nucleic acid molecule comprising the nucleotide sequence set forth in SEQ ID NO:3;
(c) a nucleic acid molecule comprising a nucleotide sequence which is at least 99% identical to the nucleotide sequence of SEQ ID NO:1 or SEQ ID NO:3;
(d) a nucleic acid molecule comprising a fragment of at least 1850 nucleotides of a nucleic acid comprising the nucleotide sequence of SEQ ID NO:1 or SEQ ID NO:3;
(e) a nucleic acid molecule which encodes a polypeptide comprising the amino acid sequence set forth in SEQ ID NO:2;
(f) a nucleic acid molecule which encodes a polypeptide comprising an amino acid sequence at least about 90% identical to the amino acid sequence of SEQ ID NO:2;
(g) a nucleic acid molecule which encodes a fragment of a polypeptide comprising at least 250 contiguous amino acid residues of the amino acid sequence of SEQ ID NO:2; and
(h) a nucleic acid molecule comprising a nucleotide sequence which is complementary to the nucleotide sequence of the nucleic acid molecule of any one of subparts (a) to (g),
   thereby identifying a compound capable of treating a pain disorder.

In a further aspect the invention provides a method for identifying a compound capable of treating a pain disorder comprising assaying the ability of the compound or agent to modulate calcium channel activity of a polypeptide selected from the group consisting of:
(a) a fragment of a polypeptide comprising the amino acid sequence of SEQ ID NO:2, wherein the fragment comprises at least 250 contiguous amino acids of SEQ ID NO:2;
(b) a polypeptide which is encoded by a nucleic acid molecule comprising a nucleotide sequence which is at least 99% identical to a nucleic acid comprising the nucleotide sequence of SEQ ID NO:1 or SEQ ID NO:3;
(c) a polypeptide comprising an amino acid sequence which is at least 90% identical to the amino acid sequence of SEQ ID NO:2; and
(d) a polypeptide comprising the amino acid sequence of SEQ ID NO:2, thereby identifying a compound capable of treating a pain disorder.
   Preferably said compound inhibits the expression of nucleic acid molecule of SEQ ID NO:1 or 3. Alternatively, said compound inhibits the activity of a polypeptide of SEQ ID NO:2.

In a further aspect the invention provides method for identifying a compound capable of modulating nociception comprising:
(a) contacting a cell with a test compound; and
(b) assaying the ability of the test compound to modulate the expression of a nucleic acid selected from the group consisting of:
   (a) a nucleic acid molecule comprising the nucleotide sequence set forth in SEQ ID NO:1;
   (b) a nucleic acid molecule comprising the nucleotide sequence set forth in SEQ ID NO:3;
   (c) a nucleic acid molecule comprising a nucleotide sequence which is at least 99% identical to the nucleotide sequence of SEQ ID NO:1 or SEQ ID NO:3;
   (d) a nucleic acid molecule comprising a fragment of at least 1850 nucleotides of a nucleic acid comprising the nucleotide sequence of SEQ ID NO:1 or SEQ ID NO:3;
   (e) a nucleic acid molecule which encodes a polypeptide comprising the amino acid sequence set forth in SEQ ID NO:2;
   (f) a nucleic acid molecule which encodes a polypeptide comprising an amino acid sequence at least about 90% identical to the amino acid sequence of SEQ ID NO:2;
   (g) a nucleic acid molecule which encodes a fragment of a polypeptide comprising at least 250 contiguous amino acid residues of the amino acid sequence of SEQ ID NO:2; and
   (h) a nucleic acid molecule comprising a nucleotide sequence which is complementary to the nucleotide sequence of the nucleic acid molecule of any one of subparts (a) to (g),
      thereby identifying a compound capable of modulating nociception.

In a further aspect the invention provides a method for identifying a compound capable of modulating nociception comprising:
(a) contacting a cell with a test compound; and
(b) assaying the ability of the test compound to modulate the activity of a polypeptide selected from the group consisting of:
   (i) a fragment of a polypeptide comprising the amino acid sequence of SEQ ID NO:2, wherein the fragment comprises at least 250 contiguous amino acids of SEQ ID NO:2;
   (ii) a polypeptide which is encoded by a nucleic acid molecule comprising a nucleotide sequence which is at least 99% identical to a nucleic acid comprising the nucleotide sequence of SEQ ID NO:1 or SEQ ID NO:3;
   (iii) a polypeptide comprising an amino acid sequence which is at least 90% identical to the amino acid sequence of SEQ ID NO:2; and
   (iv) a polypeptide comprising the amino acid sequence of SEQ ID NO:2, thereby identifying a compound capable of modulating nociception.

In one embodiment, a TLCC-2 nucleic acid molecule of the disclosure is at least 50%, 55%, 60%, 65%, 70%, 75%, 80%, 85%, 90%, 95%, 96%, 97%, 98%, 99% or more identical to the nucleotide sequence (*e*.*g*., to the entire length of the nucleotide sequence) shown in SEQ ID NO:1 or 3.
In a preferred embodiment, the isolated nucleic acid molecule includes the nucleotide sequence shown SEQ ID NO:1 or 3, or a complement thereof. In another embodiment, the nucleic acid molecule includes SEQ ID NO:3 and nucleotides 1-140 of SEQ ID NO:1. In yet another embodiment, the nucleic acid molecule includes SEQ ID NO:3 and nucleotides 1884-2095 of SEQ ID NO:1. In another preferred embodiment, the nucleic acid molecule consists of the nucleotide sequence shown in SEQ ID NO:1 or 3. In another preferred embodiment, the nucleic acid molecule includes a fragment of at least 706 nucleotides (e.g., 706 contiguous nucleotides) of the nucleotide sequence of SEQ ID NO:1 or SEQ ID NO:3, or a complement thereof.

In another embodiment, a TLCC-2 nucleic acid molecule includes a nucleotide sequence encoding a protein having an amino acid sequence sufficiently identical to the amino acid sequence of SEQ ID NO:2.. In a preferred embodiment, a TLCC-2 nucleic acid molecule includes a nucleotide sequence encoding a protein having an amino acid sequence at least 50%, 55%, 60%, 65%, 70%, 75%, 80%, 85%, 90%, 95%, 96%, 97%, 98%, 99% or more identical to the entire length of the amino acid sequence of SEQ ID NO:2..

In another preferred embodiment, an isolated nucleic acid molecule encodes the amino acid sequence of human TLCC-2. In yet another preferred embodiment, the nucleic acid molecule includes a nucleotide sequence encoding a protein having the amino acid sequence of SEQ ID NO:2. In yet another preferred embodiment, the nucleic acid molecule is at least 706 nucleotides in length. In a further preferred embodiment, the nucleic acid molecule is at least 706 nucleotides in length and encodes a protein having a TLCC-2 activity (as described herein).

Another embodiment of the disclosure features nucleic acid molecules, preferably TLCC-2 nucleic acid molecules, which specifically detect TLCC-2 nucleic acid molecules relative to nucleic acid molecules encoding non-TLCC-2 proteins. For example, in one embodiment, such a nucleic acid molecule is at least 706, 706-750, 750-800, 800-850, 850-900, 900-950, 950-1000, 1000-1050, 1050-1070, 1070-1100, 1100-1150, 1150-1200, 1200-1250, 1250-1300, 1300-1350, 1350-1400, 1400-1450, 1450-1500, 1500-1550, 1550-1600, 1600-1650, 1650-1700, 1700-1750, 1750-1800, 1800-1850, 1850-1900, 1900-1950, 1950-2000, 2000-2050, 2050-2100 or more nucleotides in length and hybridizes under stringent conditions to a nucleic acid molecule comprising the nucleotide sequence shown in SEQ ID NO:1, or a complement thereof.

In preferred embodiments, the nucleic acid molecules are at least 15 (*e*.*g*., 15 contiguous) nucleotides in length and hybridize under stringent conditions to nucleotides 1-27 of SEQ ID NO:1.

In other preferred embodiments, the nucleic acid molecule encodes a naturally occurring allelic variant of a polypeptide comprising the amino acid sequence of SEQ ID NO:2, wherein the nucleic acid molecule hybridizes to a nucleic acid molecule comprising SEQ ID NO:1 or 3 under stringent conditions.

Another embodiment of the disclosure provides an isolated nucleic acid molecule which is antisense to a TLCC-2 nucleic acid molecule, *e*.*g*., the coding strand of a TLCC-2 nucleic acid molecule.

Another aspect of this disclosure features isolated or recombinant TLCC-2 proteins and polypeptides. In one embodiment, an isolated TLCC-2 protein has one or more of the following domains: a transmembrane domain, a pore domain, and a proline rich domain. In a preferred embodiment, a TLCC-2 protein includes at least one or more of the following domains: a transmembrane domain, a pore domain, and a proline rich domain, and has an amino acid sequence at least about 50%, 55%, 60%, 65%, 70%, 75%, 80%, 85%, 90%, 95%, 96%, 97%, 98%, 99% or more identical to the amino acid sequence of SEQ ID NO:2. In another preferred embodiment, a TLCC-2 protein includes at least one transmembrane domain and has a TLCC-2 activity (as described herein).

In yet another preferred embodiment, a TLCC-2 protein includes one or more of the following domains: a transmembrane domain, a pore domain, and a proline rich domain, and is encoded by a nucleic acid molecule having a nucleotide sequence which hybridizes under stringent hybridization conditions to a nucleic acid molecule comprising the nucleotide sequence of SEQ ID NO:1 or 3.

In another embodiment, the disclosure features fragments of the protein having the amino acid sequence of SEQ ID NO:2, wherein the fragment comprises at least 18 or more amino acids (*e*.*g*., contiguous amino acids) of the amino acid sequence of SEQ ID NO:2. In another embodiment, a TLCC-2 protein has the amino acid sequence of SEQ ID NO:2.

In another embodiment, the disclosure features a TLCC-2 protein which is encoded by a nucleic acid molecule consisting of a nucleotide sequence at least about 50%, 55%, 60%, 65%, 70%, 75%, 80%, 85%, 90%, 95%, 96%, 97%, 98%, 99% or more identical to a nucleotide sequence of SEQ ID NO:1 or 3, or a complement thereof. This invention further features a TLCC-2 protein, which is encoded by a nucleic acid molecule consisting of a nucleotide sequence which hybridizes under stringent hybridization conditions to a nucleic acid molecule comprising the nucleotide sequence of SEQ ID NO:1 or 3, or a complement thereof.

The disclosure further features antibodies, such as monoclonal or polyclonal antibodies, that specifically bind proteins of the invention, preferably TLCC-2 proteins. In addition, the TLCC-2 proteins or biologically active portions thereof can be incorporated into pharmaceutical compositions, which optionally include pharmaceutically acceptable carriers.

In another aspect, the present disclosure provides a method for detecting the presence of a TLCC-2 nucleic acid molecule, protein, or polypeptide in a biological sample by contacting the biological sample with an agent capable of detecting a TLCC-2 nucleic acid molecule, protein, or polypeptide such that the presence of a TLCC-2 nucleic acid molecule, protein or polypeptide is detected in the biological sample.

In another aspect, the present disclosure provides a method for detecting the presence of TLCC-2 activity in a biological sample by contacting the biological sample with an agent capable of detecting an indicator of TLCC-2 activity such that the presence of TLCC-2 activity is detected in the biological sample.

In another embodiment, the disorder characterized by aberrant or unwanted TLCC-2 activity is a pain disorder, or a disorder characterized by misregulated pain signaling mechanisms.

In another aspect the invention provides methods for identifying a compound that binds to or modulates the activity of a TLCC-2 protein, by providing an indicator composition comprising a TLCC-2 protein having TLCC-2 activity, contacting the indicator composition with a test compound, and determining the effect of the test compound on TLCC-2 activity in the indicator composition to identify a compound that modulates the activity of a TLCC-2 protein.

Other features and advantages of the invention will be apparent from the following detailed description and claims.

### Brief Description of the Drawings

*Figure 1A-B* depicts the cDNA sequence and predicted amino acid sequence of human TLCC-2. The nucleotide sequence corresponds to nucleic acids 1 to 2095 of SEQ ID NO:1. The amino acid sequence corresponds to amino acids 1 to 580 of SEQ ID NO:2. The coding region without the 3' untranslated region of the human TLCC-2 gene is shown in SEQ ID NO:3.
*Figure* 2 depicts a structural, hydrophobicity, and antigenicity analysis of the human TLCC-2 protein.
*Figure* 3 depicts the results of a search which was performed against the MEMSAT database and which resulted in the identification of six "transmembrane domains" in the human TLCC-2 protein (SEQ ID NO:2).
*Figure 4* depicts the results of a search which was performed against the HMM database and which resulted in the identification of a "Fibronectin type III domain" in the human TLCC-2 protein (SEQ ID NO:2).
*Figure* 5 depicts the results of a search which was performed against the ProDom database and which resulted in the identification of a "31 K RNA-4 protein domain" in the human TLCC-2 protein (SEQ ID NO:2).
*Figure 6* depicts an alignment of the human TLCC-2 amino acid sequence (SEQ ID NO:2) with the amino acid sequences of human vanilloid receptor 1 and human vanilloid receptor 2 (Accession Numbers AAD26363 and AAD41724, set forth as SEQ ID NO:4 and SEQ ID NO:5, respectively), using the CLUSTAL W(1.74) alignment program.
*Figure 7A-B* depicts an alignment of the human TLCC-2 amino acid sequence (SEQ ID NO:2) with the amino acid sequence of polycystic kidney disease protein 2 from *Mus musculus* (Accession Number NP_032887, set forth as SEQ ID NO:6), using the CLUSTAL W(1.74) alignment program.
*Figure 8A-B* depicts an alignment of the human TLCC-2 amino acid sequence (SEQ ID NO:2) with the amino acid sequence of human melastatin (Accession Number AAC8000, set forth as SEQ ID NO:7), using the CLUSTAL W(1.74) alignment program.
*Figure 9* depicts the results of a search performed against the Prosite database and which resulted in the identification of four N-glycosylation sites in the amino acid sequence of human TLCC-2 (SEQ ID NO:2).
*Figure 10* is a graphic depiction of the relative levels of the human TLCC-2 mRNA expression in a human normal tissue panel, as determined using Taqman^{™} analysis.
*Figure 11* is a graphic depiction of the relative levels of the human TLCC-2 mRNA expression in a human normal tissue panel, as determined using Taqman^{™} analysis.

### Detailed Description of the Invention

The present invention is based, at least in part, on the discovery of novel molecules, referred to herein as "TRP-like calcium channel" or "TLCC-2" nucleic acid and protein molecules, which are novel members of the ion channel, *e*.*g*., calcium channel and/or vanilloid receptor, family. These novel molecules are capable of, for example, modulating an ion-channel mediated activity (*e*.*g*., a calcium channel- and/or vanilloid receptor-mediated activity) in a cell, *e*.*g*., a neuronal, skin, muscle (*e*.*g*., cardiac muscle), or liver cell.

The present invention is based also in part, on the discovery that the novel TLCC-2 molecules of the present invention are expressed in the brain at high levels, and are also expressed in the skin, the spinal cord and dorsal root ganglia (DRG). Moreover, the novel TLCC-2 molecules of the invention are upregulated in animal models of pain. The TLCC-2 molecules of the invention are involved in nociception (*e*.*g*., chemical, mechanical, or thermal nociception) and thereby modulate pain elicitation. Accordingly, the TLCC-2 molecules of the present invention act as targets for developing novel diagnostic targets and therapeutic agents to control pain and pain disorders.

As used herein, an "ion channel" includes a protein or polypeptide which is involved in receiving, conducting, and transmitting signals in an electrically excitable cell, *e*.*g*., a neuronal or muscle cell. Ion channels include vanilloid receptors, calcium channels, potassium channels, and sodium channels. As used herein, a "calcium channel" includes a protein or polypeptide which is involved in receiving, conducting, and trasmitting calcium ion-based signals in an electrically excitable cell. Calcium channels are calcium ion selective, and can determine membrane excitability (the ability of, for example, a neuronal cell to respond to a stimulus and to convert it into a sensory impulse). Calcium channels can also influence the resting potential of membranes, wave forms and frequencies of action potentials, and thresholds of excitation. Calcium channels are typically expressed in electrically excitable cells, *e*.*g*., neuronal cells, and may form heteromultimeric structures (*e*.*g*., composed of more than one type of subunit). Calcium channels may also be found in non-excitable cells (*e*.*g*., adipose cells or liver cells), where they may play a role in, *e*.*g*., signal transduction. Examples of calcium channels include the low-voltage-gated channels and the high-voltage-gated channels. Calcium channels are described in, for example, Davila et al. (1999) Annals New York Academy of Sciences 868:102-17 and McEnery, M. W. et al. (1998) J. Bioenergetics and Biomembranes 30(4): 409-418, the contents of which are incorporated herein by reference.

As used herein, a "vanilloid receptor" includes a non- selective cation channel that is structurally related to the TRP family of ion channels. Vanilloid receptors are also known as capsaisin receptors. Vanilloid receptors share several physical characteristics including an N-terminal cytoplasmic domain which contains three ankyrin repeats, six transmembrane domains, a pore-loop region located between transmembrane domains 5 and 6, and several kinase consensus sequences. Members of the vanilloid receptor (VR) family have been proposed to mediate the entry of extracellular calcium into cells, *e*.*g*., in response to the depletion of intracellular calcium stores. VRs are typically expressed in nociceptive neurons among other cells types and are directly activated by harmful heat, extracellular protons, and vanilloid compounds. VRs may also be expressed in nonsensory tissues and may mediate inflammatory rather than acute thermal pain. Vanilloid receptors are described in, for example, Caterina, M.J. (1997) Nature 389:816-824 and Caterina, M.J. (2000) Science 288:306-313).

As the TLCC-2 molecules of the present invention may modulate ion channel mediated activities (*e*.*g*., calcium channel- and/or vanilloid receptor- mediated activities), they may be useful for developing novel diagnostic and therapeutic agents for ion channel associated disorders (*e*.*g*., calcium channel and/or vanilloid receptor associated disorders).

As used herein, an "ion channel associated disorder" includes a disorder, disease or condition which is characterized by a misregulation of ion channel (*e*.*g*., calcium channel) and/or vanilloid receptor) mediated activity. For example, a "calcium channel associated disorder" includes a disorder, disease or condition which is characterized by a misregulation of calcium channel mediated activity. A "vanilloid receptor associated disorder" includes a disorder, disease or condition which is characterized by a misregulation of vanilloid receptor mediated activity. Ion channel associated disorders, *e*.*g*., calcium channel disorders and/or vanilloid receptor associated disorders, also include pain disorders. As used herein, the term "pain disorder" includes a disorder affecting pain signaling mechanisms. Pain disorders include disorders characterized by aberrant (*e*.*g*., excessive or amplified) pain. Examples of pain disorders include posttherapeutic neuralgia, diabetic neuropathy, postmastectomy pain syndrome, stump pain, reflex sympathetic dystrophy, trigeminal neuralgia, neuropathic pain, orofacial neuropathic pain, osteoarthritis, rheumatoid arthritis, fibromyalgia syndrome, tension myalgia, Guillian-Barre syndrome, Meralgia paraesthetica, burning mouth syndrome, fibrocitis, myofascial pain syndrome, idiopathic pain disorder, temporomandibular joint syndrome, atypical odontalgia, loin pain, haematuria syndrome, non-cardiac chest pain, low back pain, chronic nonspecific pain, psychogenic pain, musculoskeletal pain disorder, chronic pelvic pain, nonorganic chronic headache, tension-type headache, cluster headache, migraine, complex regional pain syndrome, vaginismus, nerve trunk pain, somatoform pain disorder, cyclical mastalgia, chronic fatigue syndrome, multiple somatization syndrome, chronic pain disorder, somatization disorder, Syndrome X, facial pain, idiopathic pain disorder, posttraumatic rheumatic pain modulation disorder (fibrositis syndrome), hyperalgesia, and Tangier disease.

As used herein, the term "pain signaling mechanisms" includes the cellular mechanisms involved in the development and regulation of pain, *e*.*g*., pain elicited by noxious chemical, mechanical, or thermal stimuli, in a subject, *e*.*g*., a mammal such as a human. In mammals, the initial detection of noxious chemical, mechanical, or thermal stimuli, a process referred to as "nociception", occurs predominantly at the peripheral terminals of specialized, small diameter sensory neurons. These sensory neurons transmit the information to the central nervous system, evoking a perception of pain or discomfort and initiating appropriate protective reflexes. The TLCC-2 molecules of the present invention may be present on these sensory neurons and, thus, may be involved in detecting these noxious chemical, mechanical, or thermal stimuli and transducing this information into membrane depolarization events. Thus, the TLCC-2 molecules, by participating in pain signaling mechanisms, may modulate pain elicitation and act as targets for developing novel diagnostic targets and therapeutic agents to control pain.

The term "family" when referring to the protein and nucleic acid molecules of the invention is intended to mean two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin, as well as other, distinct proteins of human origin or alternatively, can contain homologues of non-human origin, *e*.*g*., monkey proteins. Members of a family may also have common functional characteristics.

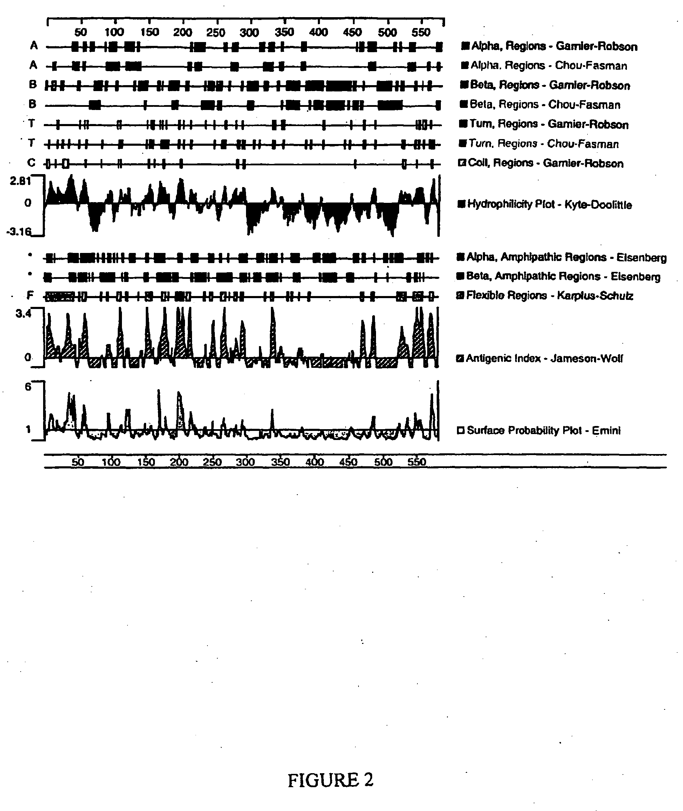

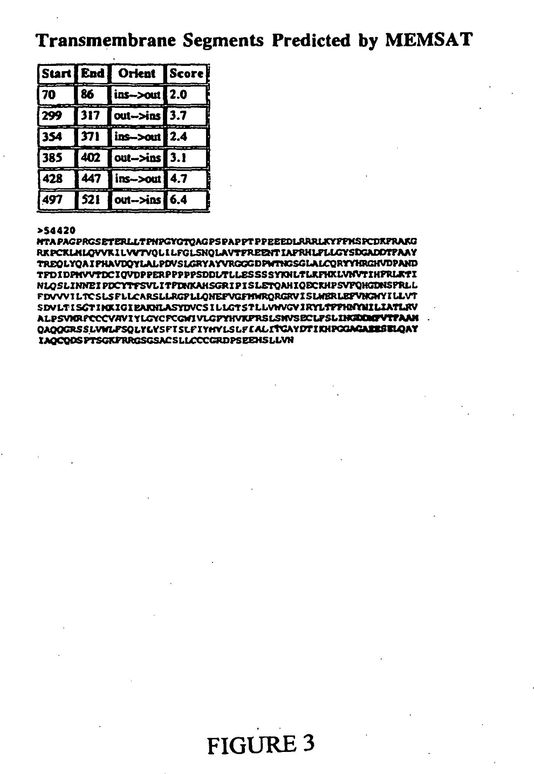

For example, the family of TLCC-2 proteins comprise at least one "transmembrane domain" and preferably six transmembrane domains. As used herein, the term "transmembrane domain" includes an amino acid sequence of about 20-45 amino acid residues in length which spans the plasma membrane. More preferably, a transmembrane domain includes about at least 20, 25, 30, 35, 40, or 45 amino acid residues and spans the plasma membrane. Transmembrane domains are rich in hydrophobic residues, and typically have an alpha-helical structure. In a preferred embodiment, at least 50%, 60%, 70%, 80%, 90%, 95% or more of the amino acids of a transmembrane domain are hydrophobic, e.g., leucines, isoleucines, tyrosines, or tryptophans. Transmembrane domains are described in, for example, Zagotta W.N. et al, (1996) Annual Rev. Neurosci. 19: 235-263, the contents of which are incorporated herein by reference. Amino acid residues 70-86, 299-317, 354-371, 385-416, 428-447, and 497-521 of the TLCC-2 protein comprise transmembrane domains (see Figures 2 and 3). Accordingly, TLCC-2 proteins having at least 50-60% homology, preferably about 60-70%, more preferably about 70-80%, or about 80-90% homology with a transmembrane domain of human TLCC-2 are within the scope of the invention.

In another embodiment, a TLCC-2 molecule of the present invention is identified based on the presence of at least one pore domain between the fifth and sixth transmembrane domains. As used herein, the term "pore domain" includes an overall hydrophobic amino acid sequence which is located between two transmembrane domains of a calcium channel protein, preferably transmembrane domains 5 and 6, and which is believed to be a major determinant of ion selectivity and channel activity in calcium channels. Pore domains are described, for example in Vannier et al. (1998) J. Biol. Chem. 273: 8675-8679 and Phillips, A. M. et al. (1992) Neuron 8, 631-642.

TLCC-2 molecules having at least one pore domain are within the scope of the invention. A pore domain may be found in the human TLCC-2 sequence (SEQ ID NO:2) at about residues 459-470 (Figure 2).

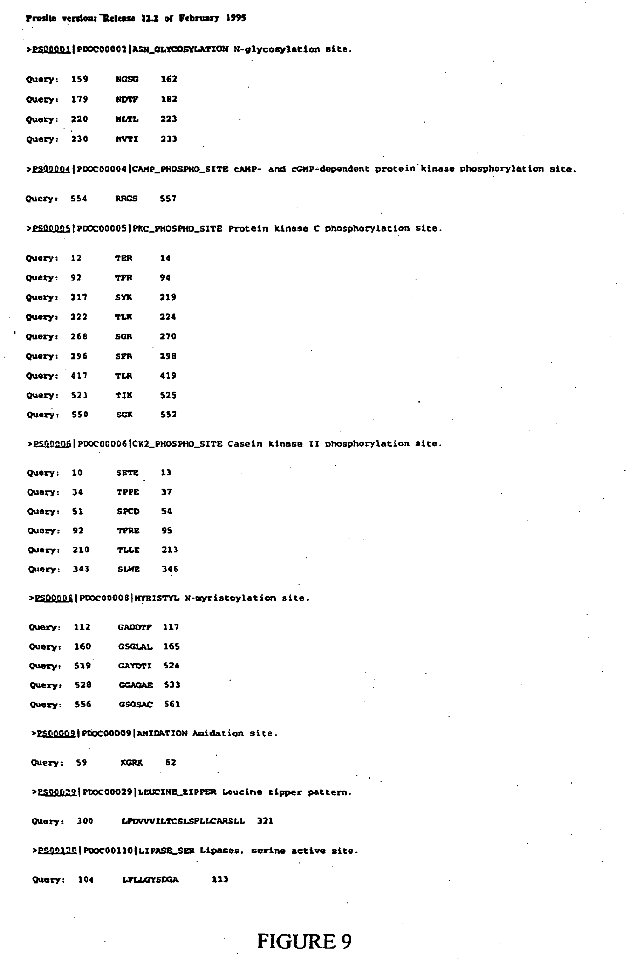

In another embodiment, a TLCC-2 molecule of the present invention is identified based on the presence of at least one N-glycosylation site. As used herein, the term "N-glycosylation site" includes an amino acid sequence of about 4 amino acid residues in length which serves as a glycosylation site. More preferably, an N-glycosylation site has the consensus sequence Asn-Xaa-Ser/Thr (where Xaa may be any amino acid) (SEQ ID NO:4). N-glycosylation sites are described in, for example, Prosite PDOC00001 (http://www.expasy.ch/cgi-bin/get-prodoc-entry?PDOC00001), the contents of which are incorporated herein by reference. Amino acid residues 159-162, 179-182, 220-223, and 230-233 of the TLCC-2 protein comprise N-glycosylation sites (see Figure 9). Accordingly, TLCC-2 proteins having at least one N-glycosylation site are within the scope of the invention.

In another embodiment, a TLCC-2 molecules of the present invention is identified based on the presence of a "proline rich domain" in the protein or corresponding nucleic acid molecule. As used herein, the term "proline rich domain" includes an amino acid sequence of about 4-6 amino acid residues in length having the general sequence X-Pro-X-X-Pro-X (where X can be any amino acid). Proline rich domains are usually located in a helical structure and bind through hydrophobic interactions to SH3 domains. SH3 domains recognize proline rich domains in both forward and reverse orientations. Proline rich domains are described in, for example, Sattler, M. et al. (1998) Leukemia 12: 637-644. Residues 1-37 of the amino acid sequence of human TLCC-2 (SEQ ID NO:2) contain proline-rich domains.

In a preferred embodiment, the TLCC-2 molecules of the invention include at least one transmembrane domain, at least one N-glycosylation site, at least one pore domain, and at least one proline rich domain.

Isolated proteins of the present invention, preferably TLCC-2 proteins, have an amino acid sequence sufficiently identical to the amino acid sequence of SEQ ID NO:2 or are encoded by a nucleotide sequence sufficiently identical to SEQ ID NO:1 or 3. As used herein, the term "sufficiently identical" refers to a first amino acid or nucleotide sequence which contains a sufficient or minimum number of identical or equivalent (*e*.*g*., an amino acid residue which has a similar side chain) amino acid residues or nucleotides to a second amino acid or nucleotide sequence such that the first and second amino acid or nucleotide sequences share common structural domains or motifs and/or a common functional activity. For example, amino acid or nucleotide sequences which share common structural domains have at least 30%, 40%, or 50% homology, preferably 60% homology, more preferably 70%-80%, and even more preferably 90-95% homology across the amino acid sequences of the domains and contain at least one and preferably two structural domains or motifs, are defined herein as sufficiently identical. Furthermore, amino acid or nucleotide sequences which share at least 30%, 40%, or 50%, preferably 60%, more preferably 70-80%, or 90-95% homology and share a common functional activity are defined herein as sufficiently identical.

As used interchangeably herein, an "TLCC-2 activity", "biological activity of TLCC-2 " or "functional activity of TLCC-2", refers to an activity exerted by a TLCC-2 protein, polypeptide or nucleic acid molecule on a TLCC-2 responsive cell or tissue, or on a TLCC-2 protein substrate, as determined *in vivo*, or *in vitro*, according to standard techniques. In one embodiment, a TLCC-2 activity is a direct activity, such as an association with a TLCC-2-target molecule. As used herein, a "target molecule" or "binding partner" is a molecule with which a TLCC-2 protein binds or interacts in nature, such that TLCC-2-mediated function is achieved. A TLCC-2 target molecule can be a non-TLCC-2 molecule or a TLCC-2 protein or polypeptide of the present invention. In an exemplary embodiment, a TLCC-2 target molecule is a TLCC-2 ligand, *e*.*g*., a calcium channel ligand. Alternatively, a TLCC-2 activity is an indirect activity, such as a cellular signaling activity mediated by interaction of the TLCC-2 protein with a TLCC-2 ligand. The biological activities of TLCC-2 are described herein. For example, the TLCC-2 proteins of the present invention can have one or more of the following activities: (1) modulate membrane excitability, (2) influence the resting potential of membranes, (3) modulate wave forms and frequencies of action potentials, (4) modulate thresholds of excitation, (5) modulate neurite outgrowth and synaptogenesis, (6) modulate signal transduction, and (7) participate in nociception.

Accordingly, another embodiment of the invention features isolated TLCC-2 proteins and polypeptides having a TLCC-2 activity. Preferred proteins are TLCC-2 proteins having at least one or more of the following domains: a transmembrane domain, an N-glycosylation site, a pore domain, and a proline rich domain, and, preferably, a TLCC-2 activity.

Additional preferred proteins have one or more of the following domains: a transmembrane domain, an N-glycosylation site, a pore domain, and a proline rich domain, and are, preferably, encoded by a nucleic acid molecule having a nucleotide sequence which hybridizes under stringent hybridization conditions to a nucleic acid molecule comprising the nucleotide sequence of SEQ ID NO:1 or 3.

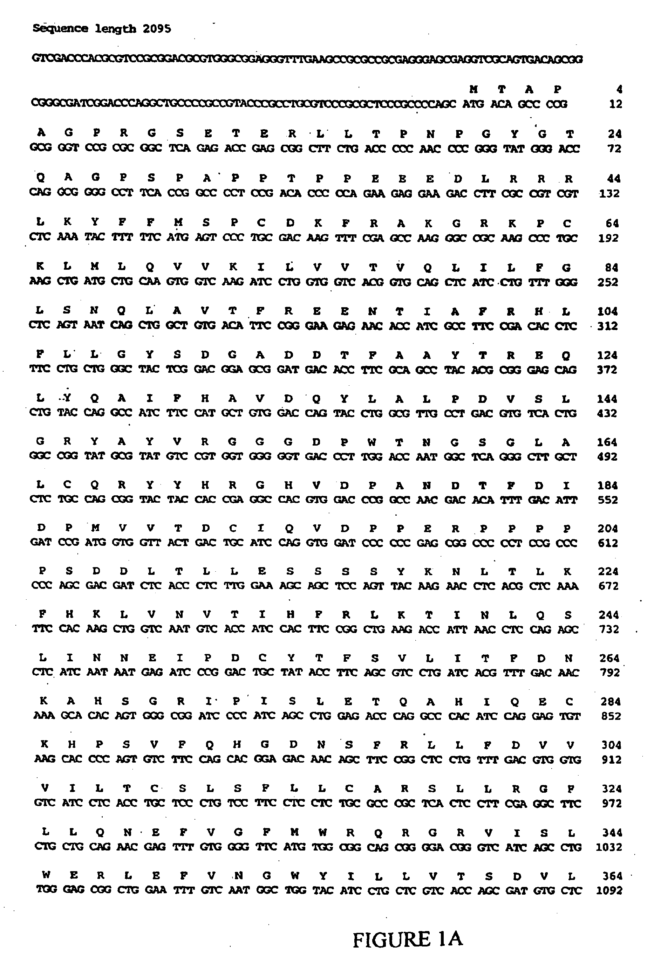

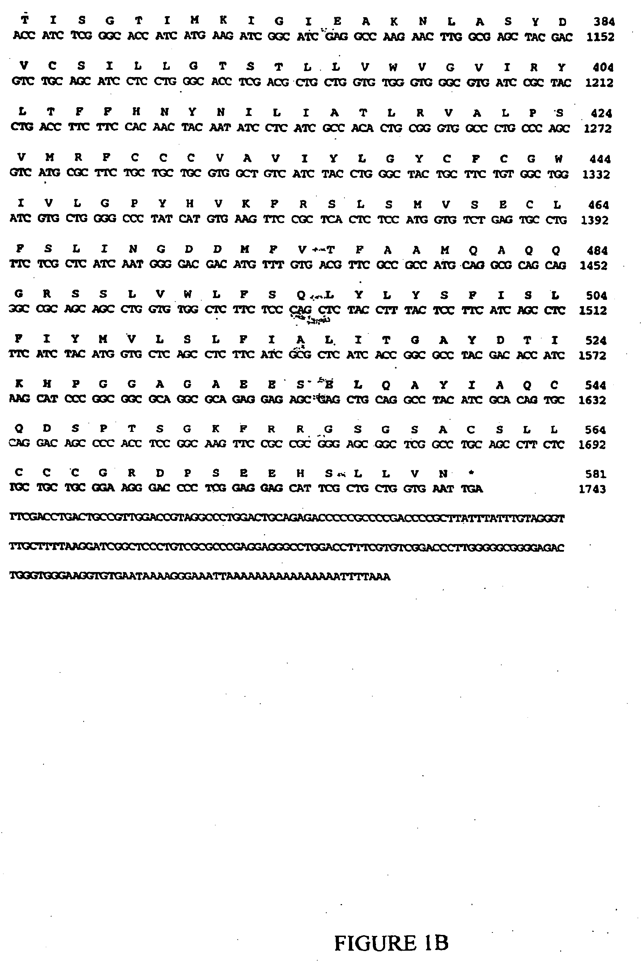

The nucleotide sequence of the isolated human TLCC-2 cDNA and the predicted amino acid sequence of the human TLCC-2 polypeptide are shown in Figure 1 and in SEQ ID NOs: I and 2, respectively. The human TLCC-2 gene, which is approximately 2095 nucleotides in length, encodes a protein having a molecular weight of approximately 65.7 kD and which is approximately 580 amino acid residues in length.

Various aspects of the invention are described in further detail in the following subsections:

### I. Isolated Nucleic Acid Molecules

One aspect of the invention pertains to isolated nucleic acid molecules that encode TLCC-2 proteins or biologically active portions thereof, as well as nucleic acid fragments sufficient for use as hybridization probes to identify TLCC-2-encoding nucleic acid molecules (*e*.*g*., TLCC-2 mRNA) and fragments for use as PCR primers for the amplification or mutation of TLCC-2 nucleic acid molecules. As used herein, the term "nucleic acid molecule" is intended to include DNA molecules (*e*.*g*., cDNA or genomic DNA) and RNA molecules (*e*.*g*., mRNA) and analogs of the DNA or RNA generated using nucleotide analogs. The nucleic acid molecule can be single-stranded or double-stranded, but preferably is double-stranded DNA.

The term "isolated nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (*i*.*e*., sequences located at the 5' and 3' ends of the nucleic acid) in the genomic DNA of the organism from which the nucleic acid is derived. For example, in various embodiments, the isolated TLCC-2 nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention, *e*.*g*., a nucleic acid molecule having the nucleotide sequence of SEQ ID NO:1 or 3, or a portion thereof, can be isolated using standard molecular biology techniques and the sequence information provided herein. Using all or a portion of the nucleic acid sequence of SEQ ID NO:1 or 3, as a hybridization probe, TLCC-2 nucleic acid molecules can be isolated using standard hybridization and cloning techniques (*e*.*g*., as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989).

Moreover, a nucleic acid molecule encompassing all or a portion of SEQ ID NO:1 or 3, can be isolated by the polymerase chain reaction (PCR) using synthetic oligonucleotide primers designed based upon the sequence of SEQ ID NO:1 or 3.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to TLCC-2 nucleotide sequences can be prepared by standard synthetic techniques, *e*.*g*., using an automated DNA synthesizer.

In a preferred embodiment, an isolated nucleic acid molecule of the invention comprises the nucleotide sequence shown in SEQ ID NO:1. The sequence of SEQ ID NO:1 corresponds to the human TLCC-2 cDNA. This cDNA comprises sequences encoding the human TLCC-2 protein (*i*.*e*., "the coding region", from nucleotides 141-1883), as well as 5' untranslated sequences (nucleotides 1-140) and 3' untranslated sequences (nucleotides 1884-2095). Alternatively, the nucleic acid molecule can comprise only the coding region of SEQ ID NO:1 (*e*.*g*., nucleotides 141-1883, corresponding to SEQ ID NO:3).

In another preferred embodiment, an isolated nucleic acid molecule of the invention comprises a nucleic acid molecule which is a complement of the nucleotide sequence shown in SEQ ID NO:1 or 3, or a portion of any of these nucleotide sequences. A nucleic acid molecule which is complementary to the nucleotide sequence shown in SEQ ID NO:1 or 3, is one which is sufficiently complementary to the nucleotide sequence shown in SEQ ID NO:1 or 3, such that it can hybridize to the nucleotide sequence shown in SEQ ID NO:1 or 3, thereby forming a stable duplex.

In still another preferred embodiment, an isolated nucleic acid molecule of the present invention comprises a nucleotide sequence which is at least about 50%, 55%, 60%, 65%, 70%, 75%, 80%, 85%, 90%, 95%, 96%, 97%, 98%, 99% or more identical to the entire length of the nucleotide sequence shown in SEQ ID NO:1 or 3, or a portion of any of these nucleotide sequences.

Moreover, the nucleic acid molecule of the invention can comprise only a portion of the nucleic acid sequence of SEQ ID NO:1 or 3, for example, a fragment which can be used as a probe or primer or a fragment encoding a portion of a TLCC-2 protein, *e*.*g*., a biologically active portion of a TLCC-2 protein. The nucleotide sequence determined from the cloning of the TLCC-2 gene allows for the generation of probes and primers designed for use in identifying and/or cloning other TLCC-2 family members, as well as TLCC-2 homologues from other species. The probe/primer typically comprises substantially purified oligonucleotide. The oligonucleotide typically comprises a region of nucleotide sequence that hybridizes under stringent conditions to at least about 12 or 15, preferably about 20 or 25, more preferably about 30, 35, 40, 45, 50, 55, 60, 65, 75, 80, 85, 90, 95, or 100 or more consecutive nucleotides of a sense sequence of SEQ ID NO:1 or 3, of an anti-sense sequence of SEQ ID NO:1 or 3, or of a naturally occurring allelic variant or mutant of SEQ ID NO:1 or 3. In one embodiment, a nucleic acid molecule of the present invention comprises a nucleotide sequence which is greater than 706, 706-750, 750-800, 800-850, 850-900, 900-950, 950-1000, 1000-1050, 1050-1070, 1070-1100, 1100-1150, 1150-1200, 1200-1250, 1250-1300, 1300-1350, 1350-1400, 1400-1450, 1450-1500, 1500-1550, 1550-1600, 1600-1650, 1650-1700, 1700-1750, 1750-1800, 1800-1850, 1850-1900, 1900-1950, 1950-2000, 2000-2050, 2050-2100 or more nucleotides in length and hybridizes under stringent hybridization conditions to a nucleic acid molecule of SEQ ID NO:1 or 3.

Probes based on the TLCC-2 nucleotide sequences can be used to detect transcripts or genomic sequences encoding the same or homologous proteins. In preferred embodiments, the probe further comprises a label group attached thereto, *e*.*g*., the label group can be a radioisotope, a fluorescent compound, an enzyme, or an enzyme co-factor. Such probes can be used as a part of a diagnostic test kit for identifying cells or tissue which misexpress a TLCC-2 protein, such as by measuring a level of a TLCC-2-encoding nucleic acid in a sample of cells from a subject *e*.*g*., detecting TLCC-2 mRNA levels or determining whether a genomic TLCC-2 gene has been mutated or deleted.

A nucleic acid fragment encoding a "biologically active portion of a TLCC-2 protein" can be prepared by isolating a portion of the nucleotide sequence of SEQ ID NO:1 or 3, which encodes a polypeptide having a TLCC-2 biological activity (the biological activities of the TLCC-2 proteins are described herein), expressing the encoded portion of the TLCC-2 protein (*e*.*g*., by recombinant expression *in vitro*) and assessing the activity of the encoded portion of the TLCC-2 protein.

The invention further encompasses nucleic acid molecules that differ from the nucleotide sequence shown in SEQ ID NO:1 or 3, due to degeneracy of the genetic code and thus encode the same TLCC-2 proteins as those encoded by the nucleotide sequence shown in SEQ ID NO:1 or 3. In another embodiment, an isolated nucleic acid molecule of the invention has a nucleotide sequence encoding a protein having an amino acid sequence shown in SEQ ID NO:2.

Accordingly, in another embodiment, an isolated nucleic acid molecule of the invention is at least 15, 20, 25, 30 or more nucleotides in length and hybridizes under stringent conditions to the nucleic acid molecule comprising the nucleotide sequence of SEQ ID NO:1 or 3. In other embodiment, the nucleic acid is at least 706, 706-750, 750-800, 800-850, 850-900, 900-950, 950-1000, 1000-1050, 1050-1070, 1070-1100, 1100-1150, 1150-1200, 1200-1250, 1250-1300, 1300-1350, 1350-1400, 1400-1450, 1450-1500, 1500-1550, 1550-1600, 1600-1650, 1650-1700, 1700-1750, 1750-1800, 1800-1850, 1850-1900, 1900-1950, 1950-2000, 2000-2050, 2050-2100 or more nucleotides in length.

As used herein, the term "hybridizes under stringent conditions" is intended to describe conditions for hybridization and washing under which nucleotide sequences that are significantly identical or homologous to each other remain hybridized to each other. Preferably, the conditions are such that sequences at least about 70%, more preferably at least about 80%, even more preferably at least about 85% or 90% identical to each other remain hybridized to each other. Such stringent conditions are known to those skilled in the art and can be found in Current Protocols in Molecular Biology, Ausubel et al., eds., John Wiley & Sons, Inc. (1995), sections 2, 4 and 6. Additional stringent conditions can be found in Molecular Cloning: A Laboratory Manual, Sambrook et al., Cold Spring Harbor Press, Cold Spring Harbor, NY (1989), chapters 7, 9 and 11. A preferred, non-limiting example of stringent hybridization conditions includes hybridization in 4X sodium chloride/sodium citrate (SSC), at about 65-70°C (or hybridization in 4X SSC plus 50% formamide at about 42-50°C) followed by one or more washes in 1X SSC, at about 65-70°C. A preferred, non-limiting example of highly stringent hybridization conditions includes hybridization in 1X SSC, at about 65-70°C (or hybridization in 1X SSC plus 50% formamide at about 42-50°C) followed by one or more washes in 0.3X SSC, at about 65-70°C. A preferred, non-limiting example of reduced stringency hybridization conditions includes hybridization in 4X SSC, at about 50-60°C (or alternatively hybridization in 6X SSC plus 50% formamide at about 40-45° C) followed by one or more washes in 2X SSC, at about 50-60°C. Ranges intermediate to the above-recited values, *e*.*g*., at 65-70°C or at 42-50°C are also intended to be encompassed by the present invention. SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes each after hybridization is complete. The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log₁₀[Na⁺]) + 0.41(%G+C) - (600/N), where N is the number of bases in the hybrid, and [Na⁺] is the concentration of sodium ions in the hybridization buffer ([Na⁺] for 1xSSC = 0.165 M). It will also be recognized by the skilled practitioner that additional reagents may be added to hybridization and/or wash buffers to decrease non-specific hybridization of nucleic acid molecules to membranes, for example, nitrocellulose or nylon membranes, including but not limited to blocking agents (*e*.*g*., BSA or salmon or herring sperm carrier DNA), detergents (*e*.*g*., SDS), chelating agents (*e*.*g*., EDTA), Ficoll, PVP and the like. When using nylon membranes, in particular, an additional preferred, non-limiting example of stringent hybridization conditions is hybridization in 0.25-0.5M NaH₂PO₄, 7% SDS at about 65°C, followed by one or more washes at 0.02M NaH₂PO₄, 1% SDS at 65°C, see *e*.*g*., Church and Gilbert (1984) Proc. Natl. Acad. Sci. USA 81:1991-1995, (or alternatively 0.2X SSC, 1% SDS).

Preferably, an isolated nucleic acid molecule of the invention that hybridizes under stringent conditions to the sequence of SEQ ID NO:1 or 3 corresponds to a naturally-occurring nucleic acid molecule. As used herein, a "naturally-occurring" nucleic acid molecule refers to an RNA or DNA molecule having a nucleotide sequence that occurs in nature (*e*.*g*., encodes a natural protein).

Accordingly, another aspect of the invention pertains to nucleic acid molecules encoding TLCC-2 proteins that contain changes in amino acid residues that are not essential for activity. Such TLCC-2 proteins differ in amino acid sequence from SEQ ID NO:2, yet retain biological activity. In one embodiment, the isolated nucleic acid molecule comprises a nucleotide sequence encoding a protein, wherein the protein comprises an amino acid sequence at least about 50%, 55%, 60%, 65%, 70%, 75%, 80%, 85%, 90%, 95%, 96%, 97%, 98%, 99% or more identical to SEQ ID NO:2.

An isolated nucleic acid molecule encoding a TLCC-2 protein identical to the protein of SEQ ID NO:2, can be created by introducing one or more nucleotide substitutions, additions or deletions into the nucleotide sequence of SEQ ID NO:1 or 3, , such that one or more amino acid substitutions, additions or deletions are introduced into the encoded protein. Mutations can be introduced into SEQ ID NO:1 or 3, by standard techniques, such as site-directed mutagenesis and PCR-mediated mutagenesis. In a preferred embodiment, a mutant TLCC-2 protein can be assayed for the ability to: (1) modulate membrane excitability, (2) influence the resting potential of membranes, (3) modulate wave forms and frequencies of action potentials, (4) modulate thresholds of excitation, (5) modulate neurite outgrowth and synaptogenesis, (6) modulate signal transduction,(7) participate in nociception, and (8) modulate pain signaling mechanisms.

In addition to the nucleic acid molecules encoding TLCC-2 proteins described above, another aspect of the invention pertains to isolated nucleic acid molecules which are antisense thereto. An "antisense" nucleic acid comprises a nucleotide sequence which is complementary to a "sense" nucleic acid encoding a protein, *e*.*g*., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire TLCC-2 coding strand, or to only a portion thereof. In one embodiment, an antisense nucleic acid molecule is antisense to a "coding region" of the coding strand of a nucleotide sequence encoding TLCC-2. The term "coding region" refers to the region of the nucleotide sequence comprising codons which are translated into amino acid residues (*e*.*g*., the coding region of human TLCC-2 corresponds to SEQ ID NO:3). In another embodiment, the antisense nucleic acid molecule is antisense to a "noncoding region" of the coding strand of a nucleotide sequence encoding TLCC-2. The term "noncoding region" refers to 5' and 3' sequences which flank the coding region that are not translated into amino acids (*i*.*e*., also referred to as 5' and 3' untranslated regions).

Given the coding strand sequences encoding TLCC-2 disclosed herein (*e*.*g*., SEQ ID NO:3), antisense nucleic acids of the invention can be designed according to the rules of Watson and Crick base pairing. The antisense nucleic acid molecule can be complementary to the entire coding region of TLCC-2 mRNA, but more preferably is an oligonucleotide which is antisense to only a portion of the coding or noncoding region of TLCC-2 mRNA. For example, the antisense oligonucleotide can be complementary to the region surrounding the translation start site of TLCC-2 mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (*e*.*g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e*.*g*., phosphorothioate derivatives and acridine substituted nucleotides can be used. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

Alternatively, TLCC-2 gene expression can be inhibited by targeting nucleotide sequences complementary to the regulatory region of the TLCC-2 (*e*.*g*., the TLCC-2 promoter and/or enhancers; *e*.*g*., nucleotides 1-137 of SEQ ID NO:1) to form triple helical structures that prevent transcription of the TLCC-2 gene in target cells. See generally, Helene, C. (1991) Anticancer Drug Des. 6(6):569-84; Helene, C. et al. (1992) Ann. N. Y. Acad. Sci. 660:27-36; and Maher, L.J. (1992) Bioassays 14(12):807-15.

In yet another embodiment, the TLCC-2 nucleic acid molecules of the present invention can be modified at the base moiety, sugar moiety or phosphate backbone to improve, *e*.*g*., the stability, hybridization, or solubility of the molecule. For example, the deoxyribose phosphate backbone of the nucleic acid molecules can be modified to generate peptide nucleic acids (see Hyrup B. et al. (1996) Bioorganic & Medicinal Chemistry 4 (1): 5-23). As used herein, the terms "peptide nucleic acids" or "PNAs" refer to nucleic acid mimics, *e*.*g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.* (1996) *supra*; Perry-O'Keefe et al. Proc. Natl. Acad. Sci. 93: 14670-675.

### II. Isolated TLCC-2 Proteins and Anti-TLCC-2 Antibodies

One aspect of the invention pertains to isolated TLCC-2 proteins, and biologically active portions thereof, as well as polypeptide fragments suitable for use as immunogens to raise anti-TLCC-2 antibodies. In one embodiment, native TLCC-2 proteins can be isolated from cells or tissue sources by an appropriate purification scheme using standard protein purification techniques. In another embodiment, TLCC-2 proteins are produced by recombinant DNA techniques. Alternative to recombinant expression, a TLCC-2 protein or polypeptide can be synthesized chemically using standard peptide synthesis techniques.

An "isolated" or "purified" protein or biologically active portion thereof is substantially free of cellular material or other contaminating proteins from the cell or tissue source from which the TLCC-2 protein is derived, or substantially free from chemical precursors or other chemicals when chemically synthesized. The language "substantially free of cellular material" includes preparations of TLCC-2 protein in which the protein is separated from cellular components of the cells from which it is isolated or recombinantly produced. In one embodiment, the language "substantially free of cellular material" includes preparations of TLCC-2 protein having less than about 30% (by dry weight) of non-TLCC-2 protein (also referred to herein as a "contaminating protein"), more preferably less than about 20% of non-TLCC-2 protein, still more preferably less than about 10% of non-TLCC-2 protein, and most preferably less than about 5% non-TLCC-2 protein. When the TLCC-2 protein or biologically active portion thereof is recombinantly produced, it is also preferably substantially free of culture medium, *i*.*e*., culture medium represents less than about 20%, more preferably less than about 10%, and most preferably less than about 5% of the volume of the protein preparation.

The language "substantially free of chemical precursors or other chemicals" includes preparations of TLCC-2 protein in which the protein is separated from chemical precursors or other chemicals which are involved in the synthesis of the protein. In one embodiment, the language "substantially free of chemical precursors or other chemicals" includes preparations of TLCC-2 protein having less than about 30% (by dry weight) of chemical precursors or non-TLCC-2 chemicals, more preferably less than about 20% chemical precursors or non-TLCC-2 chemicals, still more preferably less than about 10% chemical precursors or non-TLCC-2 chemicals, and most preferably less than about 5% chemical precursors or non-TLCC-2 chemicals.

As used herein, a "biologically active portion" of a TLCC-2 protein includes a fragment of a TLCC-2 protein which participates in an interaction between a TLCC-2 molecule and a non-TLCC-2 molecule. Biologically active portions of a TLCC-2 protein include peptides comprising amino acid sequences sufficiently identical to or derived from the amino acid sequence of the TLCC-2 protein, *e*.*g*., the amino acid sequence shown in SEQ ID NO:2, which include less amino acids than the full length TLCC-2 proteins, and exhibit at least one activity of a TLCC-2 protein. Typically, biologically active portions comprise a domain or motif with at least one activity of the TLCC-2 protein, *e*.*g*., modulating membrane excitation mechanisms. A biologically active portion of a TLCC-2 protein can be a polypeptide which is, for example, 25, 30, 35, 40, 45, 50, 75, 100, 125, 150, 175, 200, 225, 250, 275, 300, 325, 350, 375, 400, 425, 450, 274, 500, 525, 550, 575, 580, or more amino acids in length. Biologically active portions of a TLCC-2 protein can be used as targets for developing agents which modulate a TLCC-2 mediated activity, *e*.*g*., a membrane excitation mechanism.

In one embodiment, a biologically active portion of a TLCC-2 protein comprises at least one transmembrane domain. It is to be understood that a preferred biologically active portion of a TLCC-2 protein of the present invention comprises at least one or more of the following domains: a transmembrane domain, an N-glycosylation site, a pore domain, and a proline rich domain. Moreover, other biologically active portions, in which other regions of the protein are deleted, can be prepared by recombinant techniques and evaluated for one or more of the functional activities of a native TLCC-2 protein.

In a preferred embodiment, the TLCC-2 protein has an amino acid sequence shown in SEQ ID NO:2. In other embodiments, the TLCC-2 protein is substantially identical to SEQ ID NO:2, and retains the functional activity of the protein of SEQ ID NO:2, yet differs in amino acid sequence due to natural allelic variation or mutagenesis, as described in detail in subsection I above. Accordingly, in another embodiment, the TLCC-2 protein is a protein which comprises an amino acid sequence at least about 50%, 55%, 60%, 65%, 70%, 75%, 80%, 85%, 90%, 95%, 96%, 97%, 98%, 99% or more identical to SEQ ID NO:2.

To determine the percent identity of two amino acid sequences or of two nucleic acid sequences, the sequences are aligned for optimal comparison purposes (*e*.*g*., gaps can be introduced in one or both of a first and a second amino acid or nucleic acid sequence for optimal alignment and non-identical sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence (*e*.*g*., when aligning a second sequence to the TLCC-2 amino acid sequence of SEQ ID NO:2 having 580 amino acid residues, at least 50, preferably at least 100, more preferably at least 200, even more preferably at least 300, and even more preferably at least 400 or 500 or more amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (J. Mol. Biol. (48):444-453 (1970)) algorithm which has been incorporated into the GAP program in the GCG software package (available at http://www.gcg.com), using either a Blosum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (Comput. Appl. Biosci., 4:11-17 (1988)) which has been incorporated into the ALIGN program (version 2.0), using a PAM 120 weight residue table, a gap length penalty of 12 and a gap penalty of 4.

The nucleic acid and protein sequences of the present invention can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to TLCC-2 nucleic acid molecules of the invention. BLAST protein searches can be performed with the XBLAST program, score = 100, wordlength = 3, and a Blosum62 matrix to obtain amino acid sequences homologous to TLCC-2 protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e*.*g*., XBLAST and NBLAST) can be used. See http://www.ncbi.nlm.nih.gov.

In one embodiment, cell based assays can be exploited to analyze a variegated TLCC-2 library. For example, a library of expression vectors can be transfected into a cell line, *e*.*g*., an endothelial cell line, which ordinarily responds to TLCC-2 in a particular TLCC-2 substrate-dependent manner. The transfected cells are then contacted with TLCC-2 and the effect of expression of the mutant on signaling by the TLCC-2 substrate can be detected, *e*.*g*., by monitoring intracellular calcium, IP3, or diacylglycerol concentration, phosphorylation profile of intracellular proteins, or the activity of a TLCC-2-regulated transcription factor. Plasmid DNA can then be recovered from the cells which score for inhibition, or alternatively, potentiation of signaling by the TLCC-2 substrate, and the individual clones further characterized.

An isolated TLCC-2 protein, or a portion or fragment thereof, can be used as an immunogen to generate antibodies that bind TLCC-2 using standard techniques for polyclonal and monoclonal antibody preparation. A full-length TLCC-2 protein can be used or, alternatively, the invention provides antigenic peptide fragments of TLCC-2 for use as immunogens. The antigenic peptide of TLCC-2 comprises at least 8 amino acid residues of the amino acid sequence shown in SEQ ID NO:2 and encompasses an epitope of TLCC-2 such that an antibody raised against the peptide forms a specific immune complex with TLCC-2. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of TLCC-2 that are located on the surface of the protein, *e*.*g*., hydrophilic regions, as well as regions with high antigenicity (see, for example, Figure 2).

A TLCC-2 immunogen typically is used to prepare antibodies by immunizing a suitable subject, (*e*.*g*., rabbit, goat, mouse or other mammal) with the immunogen. An appropriate immunogenic preparation can contain, for example, recombinantly expressed TLCC-2 protein or a chemically synthesized TLCC-2 polypeptide. The preparation can further include an adjuvant, such as Freund's complete or incomplete adjuvant, or similar immunostimulatory agent. Immunization of a suitable subject with an immunogenic TLCC-2 preparation induces a polyclonal anti-TLCC-2 antibody response.

Accordingly, another aspect of the invention pertains to anti-TLCC-2 antibodies. The term "antibody" as used herein refers to immunoglobulin molecules and immunologically active portions of immunoglobulin molecules, *i*.*e*., molecules that contain an antigen binding site which specifically binds (immunoreacts with) an antigen, such as TLCC-2. Examples of immunologically active portions of immunoglobulin molecules include F(ab) and F(ab')2 fragments which can be generated by treating the antibody with an enzyme such as pepsin. The invention provides polyclonal and monoclonal antibodies that bind TLCC-2. The term "monoclonal antibody" or "monoclonal antibody composition", as used herein, refers to a population of antibody molecules that contain only one species of an antigen binding site capable of immunoreacting with a particular epitope of TLCC-2. A monoclonal antibody composition thus typically displays a single binding affinity for a particular TLCC-2 protein with which it immunoreacts.

Polyclonal anti-TLCC-2 antibodies can be prepared as described above by immunizing a suitable subject with a TLCC-2 immunogen. The anti-TLCC-2 antibody titer in the immunized subject can be monitored over time by standard techniques, such as with an enzyme linked immunosorbent assay (ELISA) using immobilized TLCC-2. If desired, the antibody molecules directed against TLCC-2 can be isolated from the mammal (*e*.*g*., from the blood) and further purified by well known techniques, such as protein A chromatography to obtain the IgG fraction. At an appropriate time after immunization, *e*.*g.*, when the anti-TLCC-2 antibody titers are highest, antibody-producing cells can be obtained from the subject and used to prepare monoclonal antibodies by standard techniques, such as the hybridoma technique originally described by Kohler and Milstein (1975) Nature 256:495-497) (see also, Brown el al. (1981) J. Immunol. 127:539-46; Brown et al. (1980) J. Biol. Chem .255:4980-83; Yeh et al. (1976) Proc. Natl. Acad. Sci. USA 76:2927-31; and Yeh et al. (1982) Int. J. Cancer 29:269-75), the more recent human B cell hybridoma technique (Kozbor et al. (1983) Immunol Today 4:72), the EBV-hybridoma technique (Cole et al. (1985), Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet. 3:231-36). Briefly, an immortal cell line (typically a myeloma) is fused to lymphocytes (typically splenocytes) from a mammal immunized with a TLCC-2 immunogen as described above, and the culture supernatants of the resulting hybridoma cells are screened to identify a hybridoma producing a monoclonal antibody that binds TLCC-2.

Any of the many well known protocols used for fusing lymphocytes and immortalized cell lines can be applied for the purpose of generating an anti-TLCC-2 monoclonal antibody (see, *e*.*g*., G. Galfre et al. (1977) Nature 266:55052; Gefter et al. Somatic Cell Genet., cited supra; Lerner, Yale J. Biol. Med., cited supra; Kenneth, Monoclonal Antibodies, cited supra). Moreover, the ordinarily skilled worker will appreciate that there are many variations of such methods which also would be useful. Typically, the immortal cell line (*e*.*g*., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e*.*g*., the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind TLCC-2, *e*.*g*., using a standard ELISA assay.

Alternative to preparing monoclonal antibody-secreting hybridomas, a monoclonal anti-TLCC-2 antibody can be identified and isolated by screening a recombinant combinatorial immunoglobulin library (*e*.*g*., an antibody phage display library) with TLCC-2 to thereby isolate immunoglobulin library members that bind TLCC-2. Kits for generating and screening phage display libraries are commercially available (*e*.*g*., the Pharmacia *Recombinant Phage Antibody System*, Catalog No. 27-9400-01; and the Stratagene *SurfZAP*^{™} *Phage Display Kit*, Catalog No. 240612). Additionally, examples of methods and reagents particularly amenable for use in generating and screening antibody display library can be found in, for example, Ladner et al. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J 12:725-734; Hawkins et al. (1992) J. Mol. Biol. 226:889-896; Clarkson et al. (1991) Nature 352:624-628; Gram et al. (1992) Proc. Natl. Acad. Sci. USA 89:3576-3580; Garrad et al. (1991) BiolTechnology 9:1373-1377; Hoogenboom et al. (1991) Nuc. Acid Res. 19:4133-4137; Barbas et al. (1991) Proc. Natl. Acad. Sci. USA 88:7978-7982; and McCafferty et al. Nature (1990) 348:552-554.

Additionally, recombinant anti-TLCC-2 antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson et al. International Application No. PCT/US86/02269; Akira, *et al.* European Patent Application 184,187; Taniguchi, M., European Patent Application 171,496; Morrison *et al.* European Patent Application 173,494; Neuberger et al. PCT International Publication No. WO 86/01533; Cabilly et al. U.S. Patent No. 4,816,567; Cabilly *et al.* European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. (1987) Canc. Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) BioTechniques 4:214; Winter U.S. Patent 5,225,539; Jones et al. (1986) Nature 321:552-525; Verhoeyan et al. (1988) Science 239:1534; and Beidler et al. (1988) J. Immuno/. 141:4053-4060.

An anti-TLCC-2 antibody (*e*.*g*., monoclonal antibody) can be used to isolate TLCC-2 by standard techniques, such as affinity chromatography or immunoprecipitation. An anti-TLCC-2 antibody can facilitate the purification of natural TLCC-2 from cells and of recombinantly produced TLCC-2 expressed in host cells. Moreover, an anti-TLCC-2 antibody can be used to detect TLCC-2 protein (*e*.*g*., in a cellular lysate or cell supernatant) in order to evaluate the abundance and pattern of expression of the TLCC-2 protein. Anti-TLCC-2 antibodies can be used diagnostically to monitor protein levels in tissue as part of a clinical testing procedure, *e*.*g*., to, for example, determine the efficacy of a given treatment regimen. Detection can be facilitated by coupling (*i*.*e*., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### V. Uses and Methods of the Invention

The nucleic acid molecules, proteins, protein homologues, and antibodies described herein can be used in one or more of the following methods: a) screening assays; b) predictive medicine (*e*.*g*., diagnostic assays, prognostic assays, monitoring clinical trials, and pharmacogenetics); and c) methods of treatment (*e*.*g*., therapeutic and prophylactic). As described herein, a TLCC-2 protein of the invention has one or more of the following activities: (1) modulates membrane excitability, (2) influences the resting potential of membranes, (3) modulates wave forms and frequencies of action potentials, (4) modulates thresholds of excitation, (5) modulates neurite outgrowth and synaptogenesis, (6) modulates signal transduction, and (7) participates in nociception.

The isolated nucleic acid molecules of the invention can be used, for example, to express TLCC-2 protein (*e*.*g*., via a recombinant expression vector in a host cell in gene therapy applications), to detect TLCC-2 mRNA (*e*.*g*., in a biological sample) or a genetic alteration in a TLCC-2 gene, and to modulate TLCC-2 activity, as described further below. The TLCC-2 proteins can be used to treat disorders characterized by insufficient or excessive production of a TLCC-2 substrate or production of TLCC-2 inhibitors. In addition, the TLCC-2 proteins can be used to screen for naturally occurring TLCC-2 substrates, to screen for drugs or compounds which modulate TLCC-2 activity, as well as to treat disorders characterized by insufficient or excessive production of TLCC-2 protein or production of TLCC-2 protein forms which have decreased, aberrant or unwanted activity compared to TLCC-2 wild type protein (*e*.*g*., CNS disorders (such as neurodegenerative disorders), pain disorders, or disorders of cellular growth, differentiation, or migration. Moreover, the anti-TLCC-2 antibodies of the invention can be used to detect and isolate TLCC-2 proteins, to regulate the bioavailability of TLCC-2 proteins, and modulate TLCC-2 activity.

### A. Screening Assays:

The invention provides a method (also referred to herein as a "screening assay") for identifying modulators, *i*.*e*., candidate or test compounds or agents (*e*.*g*., peptides, peptidomimetics, small molecules or other drugs) which bind to TLCC-2 proteins, have a stimulatory or inhibitory effect on, for example, TLCC-2 expression or TLCC-2 activity, or have a stimulatory or inhibitory effect on, for example, the expression or activity of TLCC-2 substrate.

In one embodiment, the invention provides assays for screening candidate or test compounds which are substrates of a TLCC-2 protein or polypeptide or biologically active portion thereof. In another embodiment, the invention provides assays for screening candidate or test compounds which bind to or modulate the activity of a TLCC-2 protein or polypeptide or biologically active portion thereof. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (*e*.*g*., Houghten (1992) Biotechniques 13:412-421), or on beads (Lam(1991) Nature 354:82-84), chips (Fodor (1993) Nature 364:555-556), bacteria (Ladner USP 5,223,409), spores (Ladner USP '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner *supra*.).

In one embodiment, an assay is a cell-based assay in which a cell which expresses a TLCC-2 protein or biologically active portion thereof is contacted with a test compound and the ability of the test compound to modulate TLCC-2 activity is determined. Determining the ability of the test compound to modulate TLCC-2 activity can be accomplished by monitoring, for example, intracellular calcium, IP3, or diacylglycerol concentration, phosphorylation profile of intracellular proteins, or the activity of a TLCC-2-regulated transcription factor. The cell, for example, can be of mammalian origin, *e*.*g*., a neuronal cell, or a liver cell.

The ability of the test compound to modulate TLCC-2 binding to a substrate or to bind to TLCC-2 can also be determined. Determining the ability of the test compound to modulate TLCC-2 binding to a substrate can be accomplished, for example, by coupling the TLCC-2 substrate with a radioisotope or enzymatic label such that binding of the TLCC-2 substrate to TLCC-2 can be determined by detecting the labeled TLCC-2 substrate in a complex. Alternatively, TLCC-2 could be coupled with a radioisotope or enzymatic label to monitor the ability of a test compound to modulate TLCC-2 binding to a TLCC-2 substrate in a complex. Determining the ability of the test compound to bind TLCC-2 can be accomplished, for example, by coupling the compound with a radioisotope or enzymatic label such that binding of the compound to TLCC-2 can be determined by detecting the labeled TLCC-2 compound in a complex. For example, compounds (*e*.*g*., TLCC-2 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a compound (*e*.*g*., a TLCC-2 substrate) to interact with TLCC-2 without the labeling of any of the interactants. For example, a microphysiometer can be used to detect the interaction of a compound with TLCC-2 without the labeling of either the compound or the TLCC-2. McConnell, H. M. et al. (1992) Science 257:1906-1912. As used herein, a "microphysiometer" (*e*.*g*., Cytosensor) is an analytical instrument that measures the rate at which a cell acidifies its environment using a light-addressable potentiometric sensor (LAPS). Changes in this acidification rate can be used as an indicator of the interaction between a compound and TLCC-2.

In another embodiment, an assay is a cell-based assay comprising contacting a cell expressing a TLCC-2 target molecule (*e*.*g*., a TLCC-2 substrate) with a test compound and determining the ability of the test compound to modulate (*e*.*g*., stimulate or inhibit) the activity of the TLCC-2 target molecule. Determining the ability of the test compound to modulate the activity of a TLCC-2 target molecule can be accomplished, for example, by determining the ability of the TLCC-2 protein to bind to or interact with the TLCC-2 target molecule.

Determining the ability of the TLCC-2 protein, or a biologically active fragment thereof, to bind to or interact with a TLCC-2 target molecule can be accomplished by one of the methods described above for determining direct binding. In a preferred embodiment, determining the ability of the TLCC-2 protein to bind to or interact with a TLCC-2 target molecule can be accomplished by determining the activity of the target molecule. For example, the activity of the target molecule can be determined by detecting induction of a cellular second messenger of the target (*i*.*e*., intracellular Ca²⁺, diacylglycerol, IP₃, and the like), detecting catalytic/enzymatic activity of the target using an appropriate substrate, detecting the induction of a reporter gene (comprising a target-responsive regulatory element operatively linked to a nucleic acid encoding a detectable marker, *e*.*g*., luciferase), or detecting a target-regulated cellular response.

In yet another embodiment, an assay of the present invention is a cell-free assay in which a TLCC-2 protein or biologically active portion thereof is contacted with a test compound and the ability of the test compound to bind to the TLCC-2 protein or biologically active portion thereof is determined. Preferred biologically active portions of the TLCC-2 proteins to be used in assays of the present invention include fragments which participate in interactions with non-TLCC-2 molecules, *e*.*g*., fragments with high surface probability scores (see, for example, Figure 2). Binding of the test compound to the TLCC-2 protein can be determined either directly or indirectly as described above. In a preferred embodiment, the assay includes contacting the TLCC-2 protein or biologically active portion thereof with a known compound which binds TLCC-2 to form an assay mixture, contacting the assay mixture with a test compound, and determining the ability of the test compound to interact with a TLCC-2 protein, wherein determining the ability of the test compound to interact with a TLCC-2 protein comprises determining the ability of the test compound to preferentially bind to TLCC-2 or biologically active portion thereof as compared to the known compound.

In another embodiment, the assay is a cell-free assay in which a TLCC-2 protein or biologically active portion thereof is contacted with a test compound and the ability of the test compound to modulate (*e*.*g*., stimulate or inhibit) the activity of the TLCC-2 protein or biologically active portion thereof is determined. Determining the ability of the test compound to modulate the activity of a TLCC-2 protein can be accomplished, for example, by determining the ability of the TLCC-2 protein to bind to a TLCC-2 target molecule by one of the methods described above for determining direct binding. Determining the ability of the TLCC-2 protein to bind to a TLCC-2 target molecule can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (*e*.*g*., BIAcore). Changes in the optical phenomenon of surface plasmon resonance (SPR) can be used as an indication of real-time reactions between biological molecules.

In an alternative embodiment, determining the ability of the test compound to modulate the activity of a TLCC-2 protein can be accomplished by determining the ability of the TLCC-2 protein to further modulate the activity of a downstream effector of a TLCC-2 target molecule. For example, the activity of the effector molecule on an appropriate target can be determined or the binding of the effector to an appropriate target can be determined as previously described.

In yet another embodiment, the cell-free assay involves contacting a TLCC-2 protein or biologically active portion thereof with a known compound which binds the TLCC-2 protein to form an assay mixture, contacting the assay mixture with a test compound, and determining the ability of the test compound to interact with the TLCC-2 protein, wherein determining the ability of the test compound to interact with the TLCC-2 protein comprises determining the ability of the TLCC-2 protein to preferentially bind to or modulate the activity of a TLCC-2 target molecule.

In more than one embodiment of the above assay methods of the present invention, it may be desirable to immobilize either TLCC-2 or its target molecule to facilitate separation of complexed from uncomplexed forms of one or both of the proteins, as well as to accommodate automation of the assay. Binding of a test compound to a TLCC-2 protein, or interaction of a TLCC-2 protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/ TLCC-2 fusion proteins or glutathione-S-transferase/target fusion proteins can be adsorbed onto glutathione sepharose beads (Sigma Chemical, St. Louis, MO) or glutathione derivatized microtitre plates, which are then combined with the test compound or the test compound and either the non-adsorbed target protein or TLCC-2 protein, and the mixture incubated under conditions conducive to complex formation (*e*.*g*., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of TLCC-2 binding or activity determined using standard techniques.

Other techniques for immobilizing proteins on matrices can also be used in the screening assays of the invention. For example, either a TLCC-2 protein or a TLCC-2 target molecule can be immobilized utilizing conjugation of biotin and streptavidin. Biotinylated TLCC-2 protein or target molecules can be prepared from biotin-NHS (N-hydroxy-succinimide) using techniques known in the art (*e*.*g*., biotinylation kit, Pierce Chemicals, Rockford, IL), and immobilized in the wells of streptavidin-coated 96 well plates (Pierce Chemical). Alternatively, antibodies reactive with TLCC-2 protein or target molecules but which do not interfere with binding of the TLCC-2 protein to its target molecule can be derivatized to the wells of the plate, and unbound target or TLCC-2 protein trapped in the wells by antibody conjugation. Methods for detecting such complexes, in addition to those described above for the GST-immobilized complexes, include immunodetection of complexes using antibodies reactive with the TLCC-2 protein or target molecule, as well as enzyme-linked assays which rely on detecting an enzymatic activity associated with the TLCC-2 protein or target molecule.

In another embodiment, modulators of TLCC-2 expression are identified in a method wherein a cell is contacted with a candidate compound and the expression of TLCC-2 mRNA or protein in the cell is determined. The level of expression of TLCC-2 mRNA or protein in the presence of the candidate compound is compared to the level of expression of TLCC-2 mRNA or protein in the absence of the candidate compound. The candidate compound can then be identified as a modulator of TLCC-2 expression based on this comparison. For example, when expression of TLCC-2 mRNA or protein is greater (statistically significantly greater) in the presence of the candidate compound than in its absence, the candidate compound is identified as a stimulator of TLCC-2 mRNA or protein expression. Alternatively, when expression of TLCC-2 mRNA or protein is less (statistically significantly less) in the presence of the candidate compound than in its absence, the candidate compound is identified as an inhibitor of TLCC-2 mRNA or protein expression. The level of TLCC-2 mRNA or protein expression in the cells can be determined by methods described herein for detecting TLCC-2 mRNA or protein.

In yet another aspect of the invention, the TLCC-2 proteins can be used as "bait proteins" in a two-hybrid assay or three-hybrid assay (see, *e*.*g*., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with TLCC-2 ("TLCC-2-binding proteins" or "TLCC-2-bp") and are involved in TLCC-2 activity. Such TLCC-2-binding proteins are also likely to be involved in the propagation of signals by the TLCC-2 proteins or TLCC-2 targets as, for example, downstream elements of a TLCC-2-mediated signaling pathway. Alternatively, such TLCC-2-binding proteins are likely to be TLCC-2 inhibitors.

The two-hybrid system is based on the modular nature of most transcription factors, which consist of separable DNA-binding and activation domains. Briefly, the assay utilizes two different DNA constructs. In one construct, the gene that codes for a TLCC-2 protein is fused to a gene encoding the DNA binding domain of a known transcription factor (*e*.*g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a TLCC-2-dependent complex, the DNA-binding and activation domains of the transcription factor are brought into close proximity. This proximity allows transcription of a reporter gene (*e*.*g*., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the TLCC-2 protein.

In another aspect, the invention pertains to a combination of two or more of the assays described herein. For example, a modulating agent can be identified using a cell-based or a cell free assay, and the ability of the agent to modulate the activity of a TLCC-2 protein can be confirmed *in vivo*, *e*.*g*., in an animal such as an animal model for cellular pain.

This invention further pertains to novel agents identified by the above-described screening assays. Accordingly, it is within the scope of this invention to further use an agent identified as described herein in an appropriate animal model. For example, an agent identified as described herein (*e*.*g*., a TLCC-2 modulating agent, an antisense TLCC-2 nucleic acid molecule, a TLCC-2-specific antibody, or a TLCC-2-binding partner) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

Models for studying pain *in vivo* include rat models of neuropathic pain caused by methods such as intraperitoneal administration of Taxol (Authier et al. (2000) Brain Res. 887:239-249), chronic constriction injury (CCI), partial sciatic nerve transection (Linenlaub and Sommer (2000) Pain 89:97-106), transection of the tibial and sural nerves (Lee et al. (2000) Neurosci. Lett. 291:29-32), the spared nerve injury model (Decosterd and Woolf (2000) Pain 87:149-158), cuffing the sciatic nerve (Pitcher and Henry (2000) Eur. J. Neurosci. 12:2006-2020), unilateral tight ligation (Esser and Sawynok (2000) Eur. J. Pharmacol. 399:131-139), L5 spinal nerve ligation (Honroe et al. (2000) Neurosci. 98:585-598), and photochemically induced ischemic nerve injury (Hao et al. (2000) Exp. Neurol. 163:231-238); rat models of nociceptive pain caused by methods such as the Chung Method, the Bennett Method, and intraperitoneal administration of complete Freund's adjuvant (CFA) (Abdi et al. (2000) Anesth. Analg. 91:955-959); rat models of post-incisional pain caused by incising the skin and fascia of a hind paw (Olivera and Prado (2000) Braz. J. Med. Biol. Res. 33:957-960); rat models of cancer pain caused by methods such as injecting osteolytic sarcoma cells into the femur (Honroe et al. (2000) Neurosci. 98:585-598); and rat models of visceral pain caused by methods such as intraperitoneal administration of cyclophosphamide.

Various methods of determining an animal's response to pain are known in the art. Examples of such methods include, but are not limited to brief intense exposure to a focused heat source, administration of a noxious chemical subcutaneously, the tail flick test, the hot plate test, the formalin test, Von Frey threshold, and testing for stress-induced analgesia (*et al*., by restraint, foot shock, and/or cold water swim) (Crawley (2000) What's Wrong With My Mouse? Wiley-Liss pp. 72-75).

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### C. Predictive Medicine:

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining TLCC-2 protein and/or nucleic acid expression as well as TLCC-2 activity, in the context of a biological sample (*e*.*g*., blood, serum, cells, tissue) to thereby determine whether an individual is afflicted with a disease or disorder, or is at risk of developing a disorder, associated with aberrant or unwanted TLCC-2 expression or activity. The invention also provides for prognostic (or predictive) assays for determining whether an individual is at risk of developing a disorder associated with TLCC-2 protein, nucleic acid expression or activity. For example, mutations in a TLCC-2 gene can be assayed in a biological sample. Such assays can be used for prognostic or predictive purpose to thereby phophylactically treat an individual prior to the onset of a disorder characterized by or associated with TLCC-2 protein, nucleic acid expression or activity.

Another aspect of the invention pertains to monitoring the influence of agents (*e*.*g*., drugs, compounds) on the expression or activity of TLCC-2 in clinical trials.

These and other agents are described in further detail in the following sections.

### 1. Diagnostic Assays

An exemplary method for detecting the presence or absence of TLCC-2 protein or nucleic acid in a biological sample involves obtaining a biological sample from a test subject and contacting the biological sample with a compound or an agent capable of detecting TLCC-2 protein or nucleic acid (*e*.*g*., mRNA, or genomic DNA) that encodes TLCC-2 protein such that the presence of TLCC-2 protein or nucleic acid is detected in the biological sample. A preferred agent for detecting TLCC-2 mRNA or genomic DNA is a labeled nucleic acid probe capable of hybridizing to TLCC-2 mRNA or genomic DNA. The nucleic acid probe can be, for example, the TLCC-2 nucleic acid set forth in SEQ ID NO:1 or 3, or a portion thereof, such as an oligonucleotide of at least 15, 30, 50, 100, 250 or 500 nucleotides in length and sufficient to specifically hybridize under stringent conditions to TLCC-2 mRNA or genomic DNA. Other suitable probes for use in the diagnostic assays of the invention are described herein.

A preferred agent for detecting TLCC-2 protein is an antibody capable of binding to TLCC-2 protein, preferably an antibody with a detectable label. Antibodies can be polyclonal, or more preferably, monoclonal. An intact antibody, or a fragment thereof (*e*.*g*., Fab or F(ab')2) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect TLCC-2 mRNA, protein, or genomic DNA in a biological sample *in vitro* as well as *in vivo*. For example, *in vitro* techniques for detection of TLCC-2 mRNA include Northern hybridizations and *in situ* hybridizations. *In vitro* techniques for detection of TLCC-2 protein include enzyme linked immunosorbent assays (ELISAs), Western blots, immunoprecipitations and immunofluorescence. *In vitro* techniques for detection of TLCC-2 genomic DNA include Southern hybridizations. Furthermore, *in vivo* techniques for detection of TLCC-2 protein include introducing into a subject a labeled anti-TLCC-2 antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample from a control subject, contacting the control sample with a compound or agent capable of detecting TLCC-2 protein, mRNA, or genomic DNA, such that the presence of TLCC-2 protein, mRNA or genomic DNA is detected in the biological sample, and comparing the presence of TLCC-2 protein, mRNA or gnomic DNA in the control sample with the presence of TLCC-2 protein, mRNA or genomic DNA in the test sample.

The invention also encompasses kits for detecting the presence of TLCC-2 in a biological sample. For example, the kit can comprise a labeled compound or agent capable of detecting TLCC-2 protein or mRNA in a biological sample; means for determining the amount of TLCC-2 in the sample; and means for comparing the amount of TLCC-2 in the sample with a standard. The compound or agent can be packaged in a suitable container. The kit can further comprise instructions for using the kit to detect TLCC-2 protein or nucleic acid.

### 2. Prognostic Assays

The diagnostic methods described herein can furthermore be utilized to identify subjects having or at risk of developing a disease or disorder associated with aberrant or unwanted TLCC-2 expression or activity. As used herein, the term "aberrant" includes a TLCC-2 expression or activity which deviates from the wild type TLCC-2 expression or activity. Aberrant expression or activity includes increased or decreased expression or activity, as well as expression or activity which does not follow the wild type developmental pattern of expression or the subcellular pattern of expression. For example, aberrant TLCC-2 expression or activity is intended to include the cases in which a mutation in the TLCC-2 gene causes the TLCC-2 gene to be under-expressed or over-expressed and situations in which such mutations result in a non-functional TLCC-2 protein or a protein which does not function in a wild-type fashion, *e*.*g*., a protein which does not interact with a TLCC-2 substrate, *e*.*g*., a non-calcium channel subunit or ligand and/or a non-vanilloid receptor subunit or ligand, or one which interacts with a non-TLCC-2 substrate, *e*.*g*. a non-calcium channel subunit or ligand and/or a non-vanilloid receptor subunit or ligand. As used herein, the term "unwanted" includes an unwanted phenomenon involved in a biological response, such as cellular proliferation. For example, the term unwanted includes a TLCC-2 expression or activity which is undesirable in a subject.

The assays described herein, such as the preceding diagnostic assays or the following assays, can be utilized to identify a subject having or at risk of developing a disorder associated with a misregulation in TLCC-2 protein activity or nucleic acid expression, such as a CNS disorder (*e*.*g*., a neurodegenerative disorder, a pain disorder, or a cellular proliferation, growth, differentiation, or migration disorder). Alternatively, the prognostic assays can be utilized to identify a subject having or at risk for developing a disorder associated with a misregulation in TLCC-2 protein activity or nucleic acid expression, such as a CNS disorder, a pain disorder, or a cellular proliferation, growth, differentiation, or migration disorder. Thus, the present invention provides a method for identifying a disease or disorder associated with aberrant or unwanted TLCC-2 expression or activity in which a test sample is obtained from a subject and TLCC-2 protein or nucleic acid (*e*.*g*., mRNA or genomic DNA) is detected, wherein the presence of TLCC-2 protein or nucleic acid is diagnostic for a subject having or at risk of developing a disease or disorder associated with aberrant or unwanted TLCC-2 expression or activity. As used herein, a "test sample" refers to a biological sample obtained from a subject of interest. For example, a test sample can be a biological fluid (*e*.*g*., serum), cell sample, *e*.*g*., neuronal cells, or tissue.

Furthermore, the prognostic assays described herein can be used to determine whether a subject can be administered an agent (*e*.*g*., an agonist, antagonist, peptidomimetic, protein, peptide, nucleic acid, small molecule, or other drug candidate) to treat a disease or disorder associated with aberrant or unwanted TLCC-2 expression or activity. For example, such methods can be used to determine whether a subject can be effectively treated with an agent for a CNS disorder, pain disorder, or a cellular proliferation, growth, differentiation, or migration disorder. Thus, the present invention provides methods for determining whether a subject can be effectively treated with an agent for a disorder associated with aberrant or unwanted TLCC-2 expression or activity in which a test sample is obtained and TLCC-2 protein or nucleic acid expression or activity is detected (*e*.*g*., wherein the abundance of TLCC-2 protein or nucleic acid expression or activity is diagnostic for a subject that can be administered the agent to treat a disorder associated with aberrant or unwanted TLCC-2 expression or activity).

The methods of the invention can also be used to detect genetic alterations in a TLCC-2 gene, thereby determining if a subject with the altered gene is at risk for a disorder characterized by misregulation in TLCC-2 protein activity or nucleic acid expression, such as a CNS disorder, pain disorder, or a disorder of cellular growth, differentiation, or migration. In preferred embodiments, the methods include detecting, in a sample of cells from the subject, the presence or absence of a genetic alteration characterized by at least one of an alteration affecting the integrity of a gene encoding a TLCC-2 -protein, or the mis-expression of the TLCC-2 gene. For example, such genetic alterations can be detected by ascertaining the existence of at least one of 1) a deletion of one or more nucleotides from a TLCC-2 gene; 2) an addition of one or more nucleotides to a TLCC-2 gene; 3) a substitution of one or more nucleotides of a TLCC-2 gene, 4) a chromosomal rearrangement of a TLCC-2 gene; 5) an alteration in the level of a messenger RNA transcript of a TLCC-2 gene, 6) aberrant modification of a TLCC-2 gene, such as of the methylation pattern of the genomic DNA, 7) the presence of a non-wild type splicing pattern of a messenger RNA transcript of a TLCC-2 gene, 8) a non-wild type level of a TLCC-2-protein, 9) allelic loss of a TLCC-2 gene, and 10) inappropriate post-translational modification of a TLCC-2-protein. As described herein, there are a large number of assays known in the art which can be used for detecting alterations in a TLCC-2 gene. A preferred biological sample is a tissue or serum sample isolated by conventional means from a subject.

In certain embodiments, detection of the alteration involves the use of a probe/primer in a polymerase chain reaction (PCR) (see, *e*.*g*., U.S. Patent Nos. 4,683,195 and 4,683,202), such as anchor PCR or RACE PCR, or, alternatively, in a ligation chain reaction (LCR) (see, *e*.*g*., Landegran et al. (1988) Science 241:1077-1080; and Nakazawa et al. (1994) Proc. Natl. Acad. Sci. USA 91:360-364), the latter of which can be particularly useful for detecting point mutations in the TLCC-2-gene (see Abravaya et al. (1995) Nucleic Acids Res .23:675-682). This method can include the steps of collecting a sample of cells from a subject, isolating nucleic acid (*e*.*g*., genomic, mRNA or both) from the cells of the sample, contacting the nucleic acid sample with one or more primers which specifically hybridize to a TLCC-2 gene under conditions such that hybridization and amplification of the TLCC-2-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., (1990) Proc. Natl. Acad. Sci. USA 87:1874-1878), transcriptional amplification system (Kwoh, D.Y. et al., (1989) Proc. Natl. Acad. Sci. USA 86:1173-1177), Q-Beta Replicase (Lizardi, P.M. et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a TLCC-2 gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in TLCC-2 can be identified by hybridizing a sample and control nucleic acids, *e*.*g*., DNA or RNA, to high density arrays containing hundreds or thousands of oligonucleotides probes (Cronin, M.T. et al. (1996) Human Mutation 7: 244-255; Kozal, M.J. et al. (1996) Nature Medicine 2: 753-759). For example, genetic mutations in TLCC-2 can be identified in two dimensional arrays containing light-generated DNA probes as described in Cronin, M.T. *et al*. *supra*. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the TLCC-2 gene and detect mutations by comparing the sequence of the sample TLCC-2 with the corresponding wild-type (control) sequence. Examples of sequencing reactions include those based on techniques developed by Maxam and Gilbert ((1977) Proc. Natl. Acad. Sci. USA 74:560) or Sanger ((1977) Proc. Natl. Acad Sci. USA 74:5463). It is also contemplated that any of a variety of automated sequencing procedures can be utilized when performing the diagnostic assays ((1995) Biotechniques 19:448), including sequencing by mass spectrometry (see, *e*.*g*., PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in the TLCC-2 gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type TLCC-2 sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl Acad Sci USA 85:4397; Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in TLCC-2 cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a TLCC-2 sequence, *e*.*g*., a wild-type TLCC-2 sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in TLCC-2 genes. For example, single strand conformation polymorphism (SSCP) may be used to detect differences in electrophoretic mobility between mutant and wild type nucleic acids (Orita et al. (1989) Proc Natl. Acad Sci USA: 86:2766, see also Cotton (1993) Mutat. Res. 285:125-144; and Hayashi (1992) Genet. Anal. Tech. Appl. 9:73-79). Single-stranded DNA fragments of sample and control TLCC-2 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5*).*

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl Acad. Sci USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e*.*g*., in clinical settings to diagnose patients exhibiting symptoms or family history of a disease or illness involving a TLCC-2 gene.

Furthermore, any cell type or tissue in which TLCC-2 is expressed may be utilized in the prognostic assays described herein.

### D. Methods of Treatment:

The present invention provides for both prophylactic and therapeutic methods of treating a subject at risk of (or susceptible to) a disorder or having a disorder associated with aberrant or unwanted TLCC-2 expression or activity, *e*.*g*. a CNS disorder, pain disorder, or a cellular proliferation, growth, differentiation, or migration disorder.

"Treatment", or "treating" as used herein, is defined as the application or administration of a therapeutic agent to a patient, or application or administration of a therapeutic agent to an isolated tissue or cell line from a patient, who has a disease or disorder, a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease. A therapeutic agent includes, but is not limited to, small molecules, peptides, antibodies, ribozymes and antisense oligonucleotides.

With regard to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (*e*.*g*., a patient's "drug response phenotype", or "drug response genotype"). Thus, another aspect of the invention provides methods for tailoring an individual's prophylactic or therapeutic treatment with either the TLCC-2 molecules of the present invention or TLCC-2 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

### 1. Prophylactic Methods

In one aspect, the invention provides a method for preventing in a subject, a disease or condition associated with an aberrant or unwanted TLCC-2 expression or activity, by administering to the subject a TLCC-2 or an agent which modulates TLCC-2 expression or at least one TLCC-2 activity. Subjects at risk for a disease which is caused or contributed to by aberrant or unwanted TLCC-2 expression or activity can be identified by, for example, any or a combination of diagnostic or prognostic assays as described herein. Administration of a prophylactic agent can occur prior to the manifestation of symptoms characteristic of the TLCC-2 aberrancy, such that a disease or disorder is prevented or, alternatively, delayed in its progression. Depending on the type of TLCC-2 aberrancy, for example, a TLCC-2, TLCC-2 agonist or TLCC-2 antagonist agent can be used for treating the subject. The appropriate agent can be determined based on screening assays described herein.

### 2. Therapeutic Methods

Another aspect of the invention pertains to methods of modulating TLCC-2 expression or activity for therapeutic purposes. Accordingly, in an exemplary embodiment, the modulatory method of the invention involves contacting a cell with a TLCC-2 or agent that modulates one or more of the activities of TLCC-2 protein activity associated with the cell. An agent that modulates TLCC-2 protein activity can be an agent as described herein, such as a nucleic acid or a protein, a naturally-occurring target molecule of a TLCC-2 protein (*e*.*g*., a TLCC-2 substrate), a TLCC-2 antibody, a TLCC-2 agonist or antagonist, a peptidomimetic of a TLCC-2 agonist or antagonist, or other small molecule. In one embodiment, the agent stimulates one or more TLCC-2 activities. Examples of such stimulatory agents include active TLCC-2 protein and a nucleic acid molecule encoding TLCC-2 that has been introduced into the cell. In another embodiment, the agent inhibits one or more TLCC-2 activities. Examples of such inhibitory agents include antisense TLCC-2 nucleic acid molecules, ribozymes, anti-TLCC-2 antibodies, and TLCC-2 inhibitors. These modulatory methods can be performed *in vitro* (*e*.*g*., by culturing the cell with the agent) or, alternatively, *in vivo* (*e*.*g*., by administering the agent to a subject). As such, the present invention provides methods of treating an individual afflicted with a disease or disorder characterized by aberrant or unwanted expression or activity of a TLCC-2 protein or nucleic acid molecule, *e*.*g*., a pain disorder. In one embodiment, the method involves administering an agent (*e*.*g*., an agent identified by a screening assay described herein), or combination of agents that modulates (*e*.*g*., upregulates or downregulates) TLCC-2 expression or activity. In another embodiment, the method involves administering a TLCC-2 protein or nucleic acid molecule as therapy to compensate for reduced, aberrant, or unwanted TLCC-2 expression or activity.

Stimulation of TLCC-2 activity is desirable in situations in which TLCC-2 is abnormally downregulated and/or in which increased TLCC-2 activity is likely to have a beneficial effect. Likewise, inhibition of TLCC-2 activity is desirable in situations in which TLCC-2 is abnormally upregulated and/or in which decreased TLCC-2 activity is likely to have a beneficial effect, *e*.*g*., in pain disorders.

### EXAMPLES

### EXAMPLE 1: IDENTIFICATION AND CHARACTERIZATION OF HUMAN TLCC-2 cDNA

In this example, the identification and characterization of the gene encoding human TLCC-2 (clone Fbh54420FL) is described.

### Isolation of the TLCC-2 cDNA

The invention is based, at least in part, on the discovery of a human gene encoding a novel protein, referred to herein as TLCC-2. The entire sequence of the human clone Fbh54420FL was determined and found to contain an open reading frame termed human "TLCC-2". The nucleotide sequence of the human TLCC-2 gene is set forth in Figures 1 and in the Sequence Listing as SEQ ID NO:1 and 3. The amino acid sequence of the human TLCC-2 expression product is set forth in Figures 1 and in the Sequence Listing as SEQ ID NO: 2.

The nucleotide sequence encoding the human TLCC-2 protein is shown in Figure 1 and is set forth as SEQ ID NO:1. The protein encoded by this nucleic acid comprises about 580 amino acids and has the amino acid sequence shown in Figure 1 and set forth as SEQ ID NO:2. The coding region (open reading frame) of SEQ ID NO:1 is set forth as SEQ ID NO:3.

### Analysis of the Human TLCC-2 Molecules

A BLASTN 2.0 search against the dbEST database, using a score of 100 and a wordlength of 12 (Altschul et al. (1990) J. Mol. Biol. 215:403) of the nucleotide sequence of human TLCC-2 revealed that human IC54420 is 99% identical to tb24a12.x1 NCI_CGAP_kid12 *Homo sapiens* cDNA clone IMAGE:2055262 3' similar to WP:R13A5.1 CE01370 (Accession Number AI307240) over nucleotides 2077 to 1377. The search further revealed that human TLCC-2 is 98% identical to au44h03.x1 Schneider fetal brain 00004 Homo sapiens cDNA clone IMAGE:2517653 3' similar to WP:R13A5.1 CE01370 (Accession Number AI816064) over nucleotides 2079-1375. This search further revealed that human TLCC-2 is 97% identical to wv36f01.x1 NCI_CGAP_Ov18 *Homo sapiens* cDNA clone IMAGE:2531641 3' similar to WP:R13A5.1 CE01370 (Accession Number AI951554) over nucleotides 2088 to 1407. This search further revealed that human TLCC-2 is 97% identical to wp80f10.x1 NCI_CGAP_Brn25 *Homo sapiens* cDNA clone IMAGE:2468107 3' similar to WP:R13A5.1 CE01370 (Accession Number AI942492) over nucleotides 2072-1422. The search further revealed that human TLCC-2 is 96% identical to nr72c11.s1 NCI_CGAP_Pr24 Homo sapiens cDNA clone IMAGE:1173524 similar to WP:R13A5.1 CE01370 (Accession Number AA641031) over nucleotides 2073-1407.

A BLASTX 2.0 search against the NRP/protot database, using a score of 100 and a wordlength of 3 (Altschul et al. (1990) J. Mol. Biol. 215:403), of the translated nucleotide sequence of human TLCC-2 revealed that human TLCC-2 is 67% or less identical to fragments (*e*.*g*., fragments of 185 amino acids or less) of unnamed protein product from *Homo sapiens* (Accession Number AK001868). The search further revealed that human TLCC-2 is 27% or less identical to fragments (*e*.*g*., fragments of 75 amino acids or less) of human polycystic kidney disease and receptor for egg jelly related protein (Accession Number AF116458). This search further revealed that human TLCC-2 is 43% or less identical to fragments (*e*.*g*., fragments of 59 amino acids or less) of polycystic kidney disease and receptor for egg jelly related protein from *Mus musculus* (Accession Number AF116459). This search further revealed that human TLCC-2 is 70% or less identical to fragments (*e*.*g*., fragments of 29 amino acids or less) of a 110 amino acid long hypothetical protein from *Aeropyrum pernix* (Accession Number AP000060) over the full length of this protein.

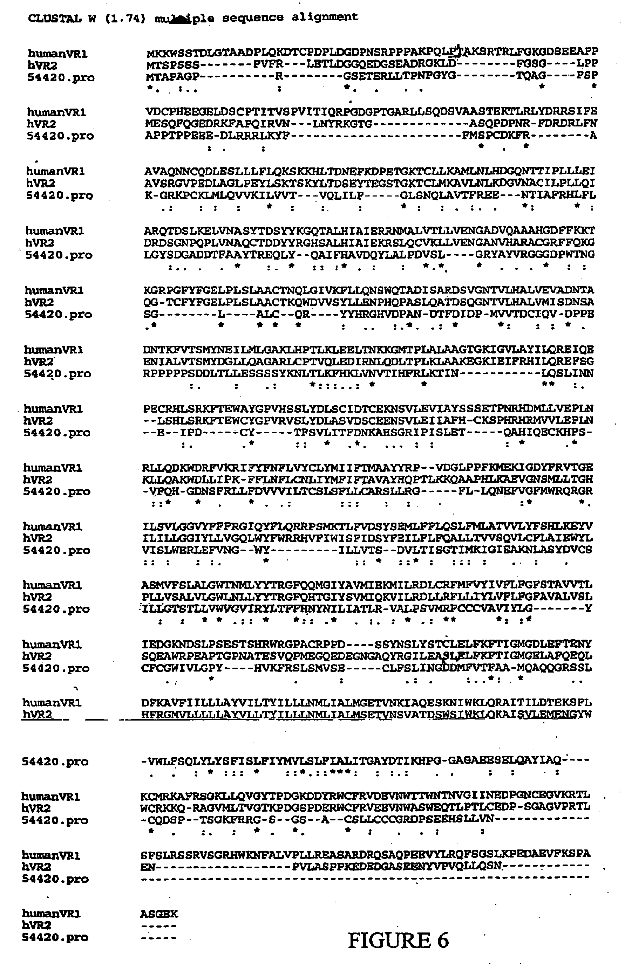

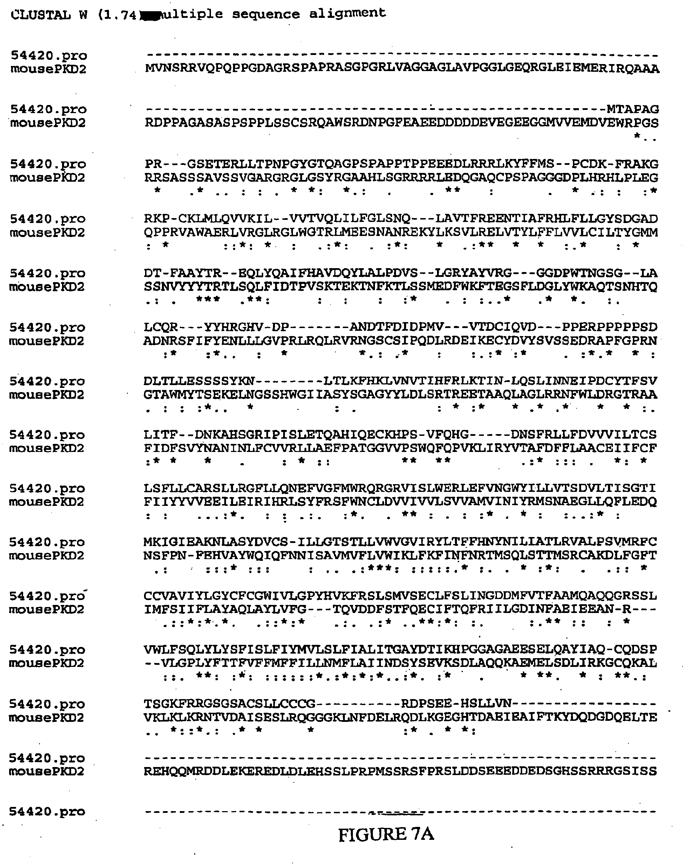

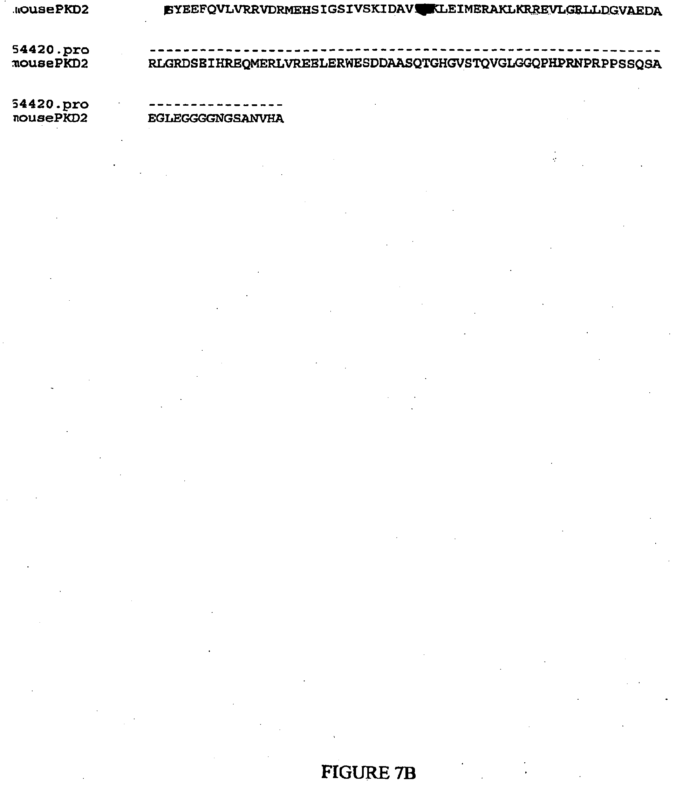

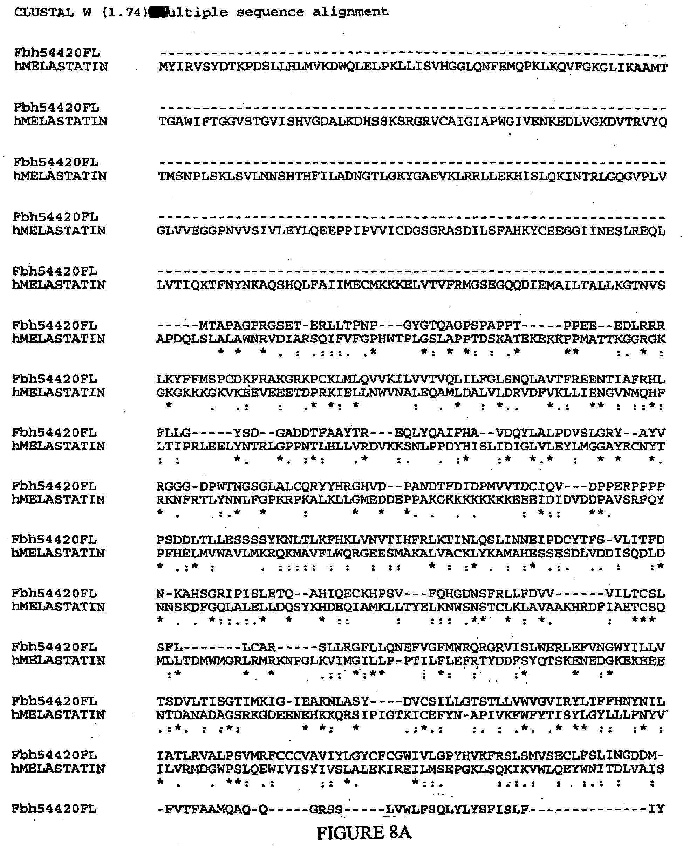

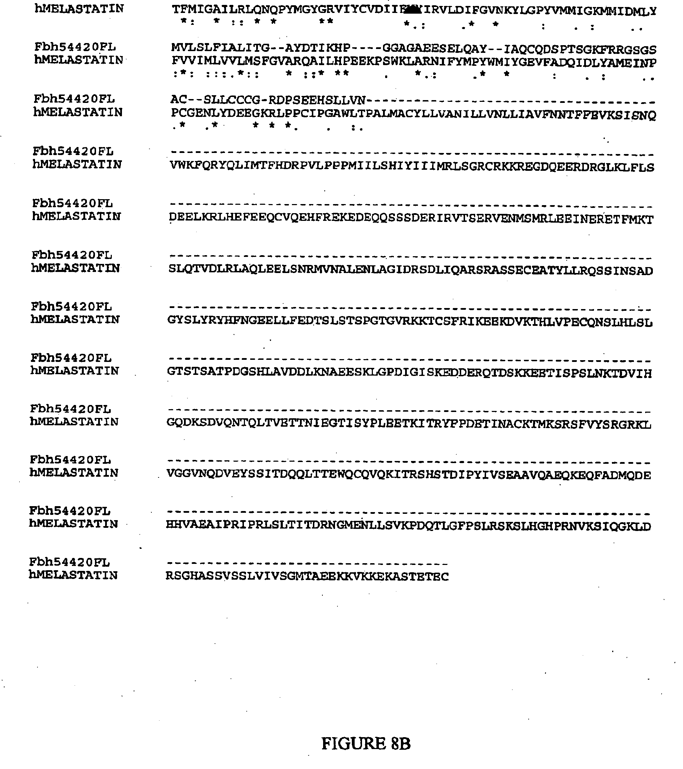

An alignment of the human TLCC-2 amino acid sequence with the amino acid sequences of vanilloid receptor I and vanilloid receptor 2 from *Homo sapiens* (Accession Numbers AAD26363 and AAD41724, SEQ ID NO:4 and SEQ ID NO:5, respectively) using the CLUSTAL W (1.74) multiple sequence alignment program is set forth in Figure 6. An alignment of the human TLCC-2 amino acid sequence with the amino acid sequence of polycystic kidney disease protein 2 from *Mus musculus* (Accession Number NP 032887, SEQ ID NO:6) using the CLUSTAL W(1.74) multiple sequence alignment program is set forth in Figure 7A-B. An alignment of the human TLCC-2 amino acid sequence with the amino acid sequence of human melastatin (Accession Number AAC8000, SEQ ID NO:7) using the CLUSTAL W(1.74) multiple sequence alignment program is set forth in Figure 8A-B.

A search was performed against the Memsat database (Figures 2 and 3), resulting in the identification of six transmembrane domains in the amino acid sequence of human TLCC-2 (SEQ ID NO:2) at about residues 70-86, 299-317, 354-371, 385-416, 428-447, and 497-521.

A search was also performed against the Prosite database (Figure 9) resulting in the identification of four N-glycosylation sites in the amino acid sequence of human TLCC-2 (SEQ ID NO:2) at about residues 159-162, 179-182, 220-223, and 230-233.

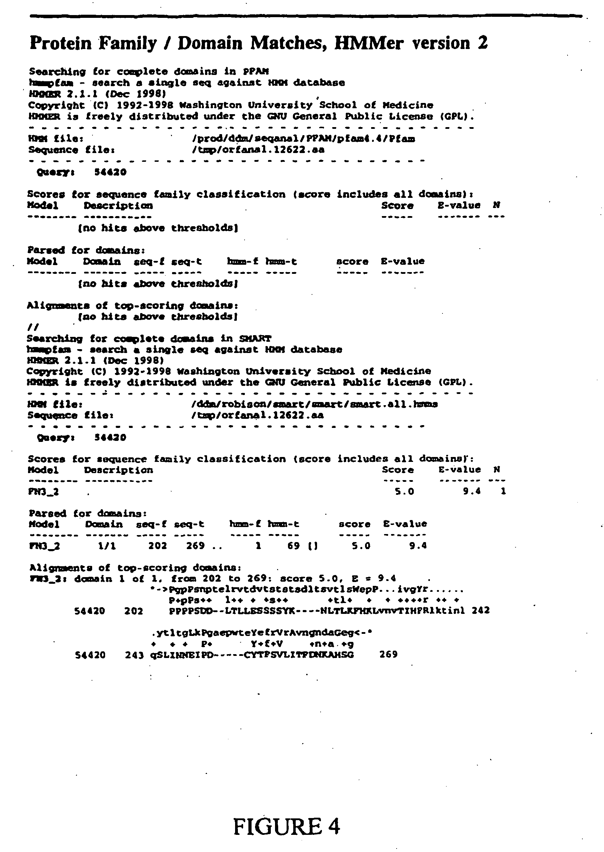

A search was also performed against the HMM database (Figure 4) resulting in the identification of a fibronectin type III domain in the amino acid sequence of human TLCC-2 (SEQ ID NO:2) at about residues 202-269 (score = 5).

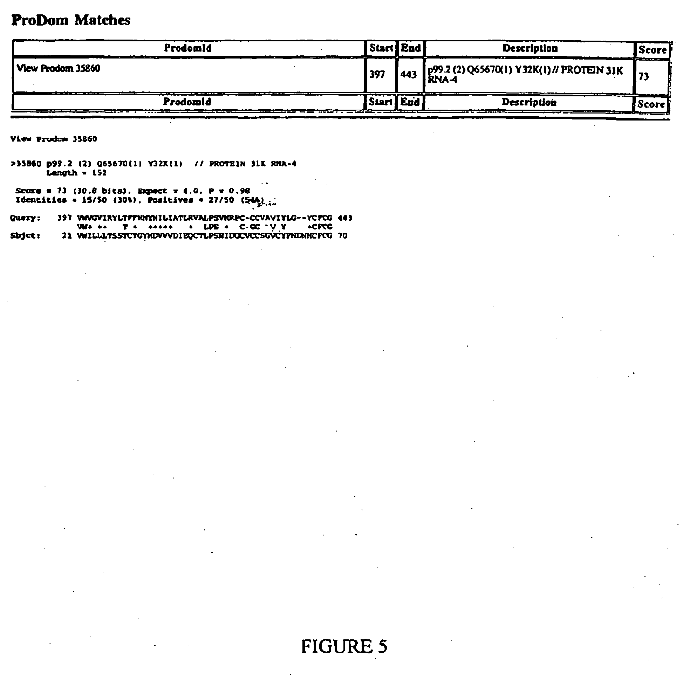

A search was also performed against the ProDom database resulting in the identification of a 31 K RNA-4 protein domain in the amino acid sequence of human TLCC-2 (SEQ ID NO:2) at about residues 397-443 (score = 73). The results of the search are set forth in Figure 5.

### Tissue Distribution of Human TLCC-2 mRNA by Northern Analysis

This example describes the tissue distribution of TLCC-2 mRNA, as determined by Northern analysis.

Northern blot hybridizations with the various RNA samples are performed under standard conditions and washed under stringent conditions, *i*.*e*., 0.2xSSC at 65°C. The DNA probe is radioactively labeled with ³²P-dCTP using the Prime-It kit (Stratagene, La Jolla, CA) according to the instructions of the supplier. Filters containing human mRNA (MultiTissue Northern I and MultiTissue Northern II from Clontech, Palo Alto, CA) are probed in ExpressHyb hybridization solution (Clontech) and washed at high stringency according to manufacturer's recommendations.

### Tissue Distribution of TLCC-2 mRNA by In situ Analysis

For *in situ* analysis, various tissues, *e*.*g*. tissues obtained from brain and spinal cord from monkey and rat, were first frozen on dry ice. Ten-micrometer-thick sections of the tissues were post-fixed with 4% formaldehyde in DEPC treated 1X phosphate-buffered saline at room temperature for 10 minutes before being rinsed twice in DEPC 1X phosphate-buffered saline and once in 0.1 M triethanolamine-HCl (pH 8.0). Following incubation in 0.25% acetic anhydride-0.1 M triethanolamine-HCl for 10 minutes, sections were rinsed in DEPC 2X SSC (1X SSC is 0.15M NaCl plus 0.015M sodium citrate). Tissue was then dehydrated through a series of ethanol washes, incubated in 100% chloroform for 5 minutes, and then rinsed in 100% ethanol for 1 minute and 95% ethanol for 1 minute and allowed to air dry.

Hybridizations were performed with ³⁵S-radiolabeled (5 X 10⁷ cpm/ml) cRNA probes. Probes were incubated in the presence of a solution containing 600 mM NaCl, 10 mM Tris (pH 7.5), 1 mM EDTA, 0.01% sheared salmon sperm DNA, 0.01% yeast tRNA, 0.05% yeast total RNA type X1, 1X Denhardt's solution, 50% formamide, 10% dextran sulfate, 100 mM dithiothreitol, 0.1% sodium dodecyl sulfate (SDS), and 0.1% sodium thiosulfate for 18 hours at 55°C.

After hybridization, slides were washed with 2X SSC. Sections are then sequentially incubated at 37°C in TNE (a solution containing 10 mM Tris-HCl (pH 7.6), 500 mM NaCl, and 1 mM EDTA), for 10 minutes, in TNE with 10µg of RNase A per ml for 30 minutes, and finally in TNE for 10 minutes. Slides were then rinsed with 2X SSC at room temperature, washed with 2X SSC at 50°C for 1 hour, washed with 0.2X SSC at 55°C for 1 hour, and 0.2X SSC at 60°C for 1 hour. Sections were then dehydrated rapidly through serial ethanol-0.3 M sodium acetate concentrations before being air dried and exposed to Kodak Biomax MR scientific imaging film for 24 hours and subsequently dipped in NB-2 photoemulsion and exposed at 4°C for 7 days before being developed and counter stained.

*In situ* hybridization results showed expression in monkey and rat brain (including cortex, stratium, and hippocampus), spinal cord, and dorsal root ganglia (DRG) neurons.

*Is situ* hybridization in rat animal models showed up-regulation of the TLCC-2 gene 10 days after unilateral chronic constriction injury (CCI). There was up-regulation of TLCC-2 seven days after axotomy and after intraplantar injection of complete Freund's adjuvant (CFA). These levels decreased to normal levels at later time points. No contralateral effects were observed. These results indicate that the TLCC-2 molecules of the present invention are up-regulated in response to painful simuli, and are therefore involved in nociception. Modulation, *e*.*g*., inhibition, of expression or activity of the TLCC-2 molecules of the invention may therefore modulate nociception and provide treatment for pain disorders.

### Tissue Expression Analysis of TLCC-2 mRNA Using Taqman Analysis

This example describes the tissue distribution of human TLCC-2 mRNA in a variety of cells and tissues, as determined using the TaqMan™ procedure. The Taqman^{™} procedure is a quantitative, reverse transcription PCR-based approach for detecting mRNA. The RT-PCR reaction exploits the 5' nuclease activity of AmpliTaq Gold^{™} DNA Polymerase to cleave a TaqMan™ probe during PCR. Briefly, cDNA was generated from the samples of interest, *e*.*g*., human brain, spinal cord, heart, kidney, liver, lung, dorsal root ganglia, and skin, and used as the starting material for PCR amplification. In addition to the 5' and 3' gene-specific primers, a gene-specific oligonucleotide probe (complementary to the region being amplified) was included in the reaction (*i*.*e*., the Taqman^{™} probe). The TaqMan™ probe includes the oligonucleotide with a fluorescent reporter dye covalently linked to the 5' end of the probe (such as FAM (6-carboxyfluorescein), TET (6-carboxy-4,7,2',7'-tetrachlorofluorescein), JOE (6-carboxy-4,5-dichloro-2,7-dimethoxyfluorescein), or VIC) and a quencher dye (TAMRA (6-carboxy-N,N,N',N'-tetramethylrhodamine) at the 3' end of the probe.

During the PCR reaction, cleavage of the probe separates the reporter dye and the quencher dye, resulting in increased fluorescence of the reporter. Accumulation of PCR products is detected directly by monitoring the increase in fluorescence of the reporter dye. When the probe is intact, the proximity of the reporter dye to the quencher dye results in suppression of the reporter fluorescence. During PCR, if the target of interest is present, the probe specifically anneals between the forward and reverse primer sites. The 5'-3' nucleolytic activity of the AmpliTaq™ Gold DNA Polymerase cleaves the probe between the reporter and the quencher only if the probe hybridizes to the target. The probe fragments are then displaced from the target, and polymerization of the strand continues. The 3' end of the probe is blocked to prevent extension of the probe during PCR. This process occurs in every cycle and does not interfere with the exponential accumulation of product. RNA was prepared using the trizol method and treated with DNase to remove contaminating genomic DNA. cDNA was synthesized using standard techniques. Mock cDNA synthesis in the absence of reverse transcriptase resulted in samples with no detectable PCR amplification of the control gene confirms efficient removal of genomic DNA contamination.

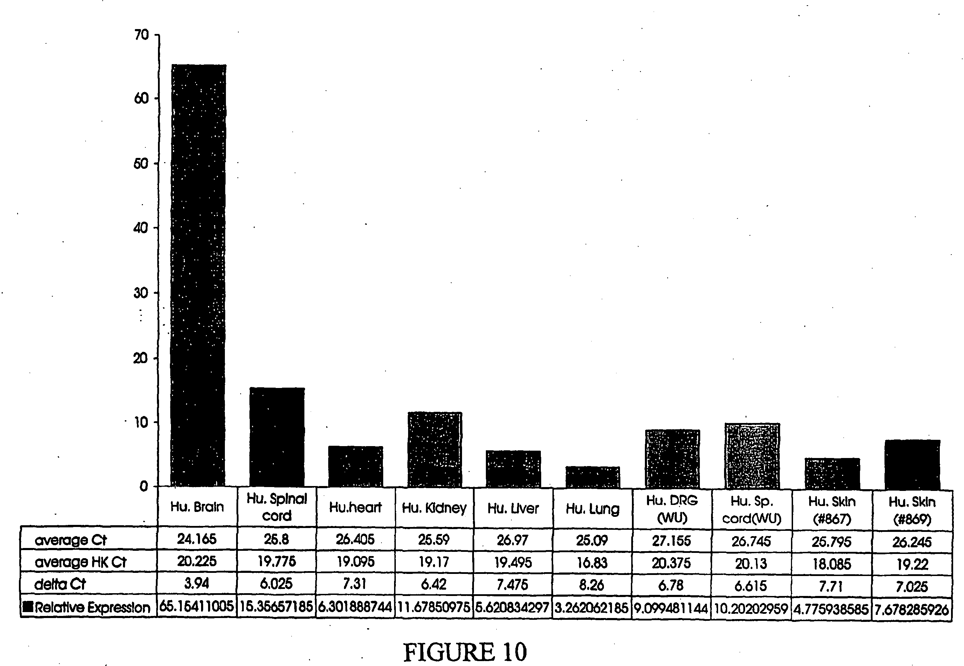

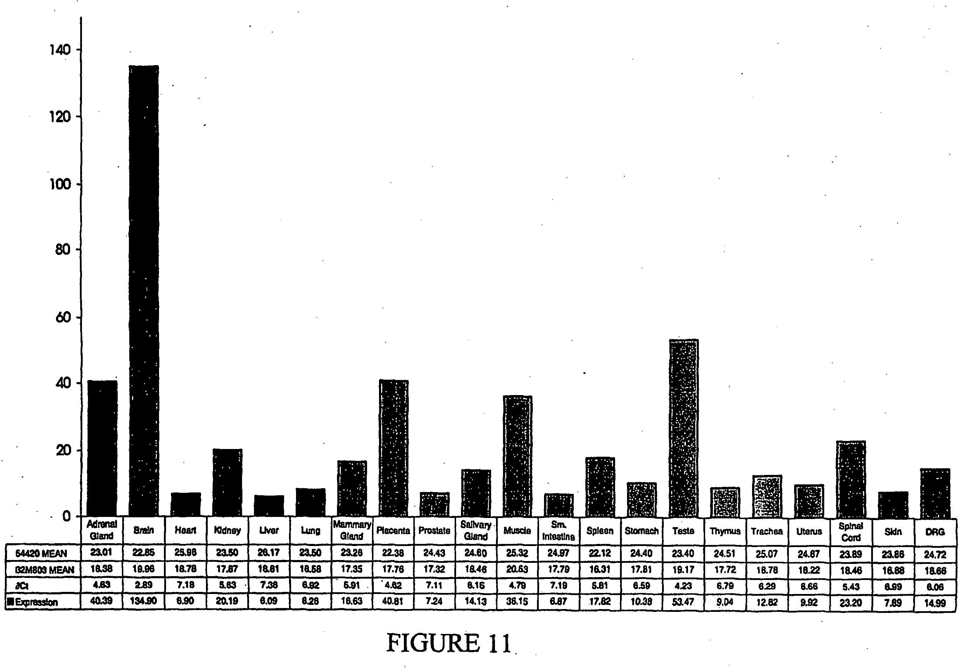

Two human normal tissue panels indicated broad distribution of human TLCC-2 expression, with highest expression in human brain, followed by testis, placenta, adrenal gland, spinal cord, skin, and dorsal root ganglia (DRG) (See Figures 10 and 11).

### EXAMPLE 2: EXPRESSION OF RECOMBINANT IC54420 PROTEIN IN BACTERIAL CELLS

In this example, TLCC-2 is expressed as a recombinant glutathione-S-transferase (GST) fusion polypeptide in *E*. *coli* and the fusion polypeptide is isolated and characterized. Specifically, TLCC-2 is fused to GST and this fusion polypeptide is expressed in *E*. *coli*, *e*.*g*., strain PEB199. Expression of the GST-TLCC-2 fusion protein in PEB199 is induced with IPTG. The recombinant fusion polypeptide is purified from crude bacterial lysates of the induced PEB199 strain by affinity chromatography on glutathione beads. Using polyacrylamide gel electrophoretic analysis of the polypeptide purified from the bacterial lysates, the molecular weight of the resultant fusion polypeptide is determined.

### EXAMPLE 3: EXPRESSION OF RECOMBINANT IC54420 PROTEIN IN COS CELLS

To express the TLCC-2 gene in COS cells, the pcDNA/Amp vector by Invitrogen Corporation (San Diego, CA) is used. This vector contains an SV40 origin of replication, an ampicillin resistance gene, an *E*. *coli* replication origin, a CMV promoter followed by a polylinker region, and an SV40 intron and polyadenylation site. A DNA fragment encoding the entire TLCC-2 protein and an HA tag (Wilson *et al*. (1984) *Cell* 37:767) or a FLAG tag fused in-frame to its 3' end of the fragment is cloned into the polylinker region of the vector, thereby placing the expression of the recombinant protein under the control of the CMV promoter.

To construct the plasmid, the TLCC-2 DNA sequence is amplified by PCR using two primers. The 5' primer contains the restriction site of interest followed by approximately twenty nucleotides of the TLCC-2 coding sequence starting from the initiation codon; the 3' end sequence contains complementary sequences to the other restriction site of interest, a translation stop codon, the HA tag or FLAG tag and the last 20 nucleotides of the TLCC-2 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the TLCC-2 gene is inserted in the correct orientation. The ligation mixture is transformed into *E*. *coli* cells (strains HB101, DH5□, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the TLCC-2-pcDNA/Amp plasmid DNA using the calcium phosphate or calcium chloride co-precipitation methods, DEAE-dextran-mediated transfection, lipofection, or electroporation. Other suitable methods for transfecting host cells can be found in Sambrook, J., Fritsh, E. F., and Maniatis, T. Molecular Cloning: A Laboratory Manual. 2nd, ed, Cold Spring Harbor Laboratory, Cold Spring Harbor Laboratory Press, Cold Spring Harbor, NY, 1989. The expression of the IC54420 polypeptide is detected by radiolabelling (³⁵S-methionine or ³⁵S-cysteine available from NEN, Boston, MA, can be used) and immunoprecipitation (Harlow, E. and Lane, D. Antibodies: A Laboratory Manual, Cold Spring Harbor Laboratory Press, Cold Spring Harbor, NY, 1988) using an HA specific monoclonal antibody. Briefly, the cells are labelled for 8 hours with ³⁵S-methionine (or ³⁵S-cysteine). The culture media are then collected and the cells are lysed using detergents (RIPA buffer, 150 mM NaCl, 1% NP-40, 0.1% SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the TLCC-2 coding sequence is cloned directly into the polylinker of the pCDNA/Amp vector using the appropriate restriction sites. The resulting plasmid is transfected into COS cells in the manner described above, and the expression of the TLCC-2 polypeptide is detected by radiolabelling and immunoprecipitation using a TLCC-2-specific monoclonal antibody.

### EXAMPLE 4: REGULATION OF CALCIUM INFLUX THROUGH TLCC-2

This experiment describes the regulation of calcium influx though TLCC-2 in HEK293 cells as determined by Fluorometric Imaging Plate Reader experiments (FLIPR) (Molecular Devices Corp., Sunnyvale, CA).

The FLIPR is a screening tool for cell-based fluorescent assays which allows the simultaneous stimulation and measurement of separate cell populations in a high throughput format. Therefore, using this system, it is possible to quantify transient signals, such as the release of intracellular calcium, from cell populations, in parallel and in real time. The FLIPR contains chambers in which to hold the test plate and plates containing antagonists or agonists to be added to the test plate. The FLIPR utilizes an argon laser that provides discrete spectral lines spaced from approximately 350 to 530 nm. For use with fluorescent Ca²⁺ dyes, the 88-nm line of the laser is employed. The laser simultaneously illuminates the wells in a test plate. The image of each well in the plate is captured by a cooled charge coupled device (CCD) camera, which updates images once per second, if required, for the measurement of rapid calcium responses. Because both excitation and emission are read via the bottom of the plate, black-walled, transparent bottomed 96-well plates are used. Data captured by the CCD camera is converted to digital data and then transferred to a computer.

Briefly, a calcium indicator (*e*.*g*., fluo-3/AM or Calcium Green-1/AM) was transferred to the culture medium. Because the FLIPR collects fluorescence from the bottom of the well, suspension cells require centrifugation to the base of the well following dye loading. Viable HEK293 cells were resuspended in loading medium and incubated for one hour. The cells were then centrifuged and resuspended with wash buffer. The cell suspension containing the dye was then aliquotted into each well of the black-walled, transparent bottomed 96-well plate and the plate was centrifuged. The FLIPR assay was then carried out and the results analyzed. (If adherent cells are used, they may be plated at an appropriate density in the 96-well plates and cultured overnight. Dye may then be loaded and incubated).

Results show a constitutive calcium influx through TLCC-2 in HEK293 cells that were incubated with NMDG/0 Ca⁺² and stimulated afterwards with 5mM Ca⁺².

## Claims

1. A method for identifying a compound which binds to a polypeptide or modulates the ability of a polypeptide to transport calcium across a membrane, wherein the polypeptide is selected from the group consisting of:
(i) a fragment of a polypeptide comprising the amino acid sequence of SEQ ID NO:2, wherein the fragment comprises at least 250 contiguous amino acids of SEQ ID NO:2;
(ii) a polypeptide which is encoded by a nucleic acid molecule comprising a nucleotide sequence which is at least 99% identical to a nucleic acid comprising the nucleotide sequence of SEQ ID NO:1 or SEQ ID NO:3;
(iii) a polypeptide comprising an amino acid sequence which is at least 90% identical to the amino acid sequence of SEQ ID NO:2; and
(iv) a polypeptide comprising the amino acid sequence of SEQ ID NO:2, comprising:
(a) contacting the polypeptide, or a cell expressing the polypeptide with a test compound; and
(b) determining whether the compound binds to the polypeptide or modulates the ability of the polypeptide to transport calcium across a membrane, thereby identifying a compound which binds to the polypeptide or modulates the ability of the polypeptide to transport calcium across a membrane.

2. A method of identifying a nucleic acid molecule associated with a pain disorder comprising:
(a) contacting a sample comprising nucleic acid molecules with a hybridization probe comprising at least 25 contiguous nucleotides of SEQ ID NO:1; and
(b) detecting the presence of a nucleic acid molecule in said sample that hybridizes to said probe, thereby identifying a nucleic acid molecule associated with a pain disorder.

3. A method of identifying a nucleic acid associated with a pain disorder comprising:
(a) contacting a sample comprising nucleic acid molecules with a first and a second amplification primer, said first primer comprising at least 25 contiguous nucleotides of SEQ ID NO:1 and said second primer comprising at least 25 contiguous nucleotides from the complement of SEQ ID NO:1;
(b) incubating said sample under conditions that allow nucleic acid amplification; and
(c) detecting the presence of a nucleic acid molecule in said sample that is amplified, thereby identifying a nucleic acid molecule associated with a pain disorder.

4. A method of identifying a polypeptide associated with a pain disorder comprising:
(a) contacting a sample comprising polypeptides with an antibody that selectively binds to a polypeptide of SEQ ID NO:2; and
(b) detecting the presence of a polypeptide in said sample that binds to said antibody, thereby identifying a polypeptide associated with a pain disorder.

5. The method of claim 4, wherein said antibody is detectably labeled.

6. A method for identifying a compound capable of treating a pain disorder comprising assaying the ability of the compound to modulate the expression of a nucleic acid molecule selected from from the group consisting of:
(a) a nucleic acid molecule comprising the nucleotide sequence set forth in SEQ ID NO:1;
(b) a nucleic acid molecule comprising the nucleotide sequence set forth in SEQ ID NO:3;
(c) a nucleic acid molecule comprising a nucleotide sequence which is at least 99% identical to the nucleotide sequence of SEQ ID NO:1 or SEQ ID NO:3;
(d) a nucleic acid molecule comprising a fragment of at least 1850 nucleotides of a nucleic acid comprising the nucleotide sequence of SEQ ID NO:1 or SEQ ID NO:3;
(e) a nucleic acid molecule which encodes a polypeptide comprising the amino acid sequence set forth in SEQ ID NO:2;
(f) a nucleic acid molecule which encodes a polypeptide comprising an amino acid sequence at least about 90% identical to the amino acid sequence of SEQ ID NO:2;
(g) a nucleic acid molecule which encodes a fragment of a polypeptide comprising at least 250 contiguous amino acid residues of the amino acid sequence of SEQ ID NO:2; and
(h) a nucleic acid molecule comprising a nucleotide sequence which is complementary to the nucleotide sequence of the nucleic acid molecule of any one of subparts (a) to (g),
thereby identifying a compound capable of treating a pain disorder.

7. A method for identifying a compound capable of treating a pain disorder comprising assaying the ability of the compound to modulate calcium channel activity of a polypeptide selected from the group consisting of:
(a) a fragment of a polypeptide comprising the amino acid sequence of SEQ ID NO:2, wherein the fragment comprises at least 250 contiguous amino acids of SEQ ID NO:2;
(b) a polypeptide which is encoded by a nucleic acid molecule comprising a nucleotide sequence which is at least 99% identical to a nucleic acid comprising the nucleotide sequence of SEQ ID NO:1 or SEQ ID NO:3;
(c) a polypeptide comprising an amino acid sequence which is at least 90% identical to the amino acid sequence of SEQ ID NO:2; and
(d) a polypeptide comprising the amino acid sequence of SEQ ID NO:2, thereby identifying a compound capable of treating a pain disorder.

8. The method of claim 6, wherein said compound inhibits the expression of nucleic acid molecule of SEQ ID NO:1 or 3.

9. The method of claim 7, wherein said compound inhibits the activity of a polypeptide of SEQ ID NO:2.

10. A method for identifying a compound capable of modulating nociception comprising:
(a) contacting a cell with a test compound; and
(b) assaying the ability of the test compound to modulate the expression of a nucleic acid selected from the group consisting of:
(a) a nucleic acid molecule comprising the nucleotide sequence set forth in SEQ ID NO:1;
(b) a nucleic acid molecule comprising the nucleotide sequence set forth in SEQ ID NO:3;
(c) a nucleic acid molecule comprising a nucleotide sequence which is at least 99% identical to the nucleotide sequence of SEQ ID NO:1 or SEQ ID NO:3;
(d) a nucleic acid molecule comprising a fragment of at least 1850 nucleotides of a nucleic acid comprising the nucleotide sequence of SEQ ID NO:1 or SEQ ID NO:3;
(e) a nucleic acid molecule which encodes a polypeptide comprising the amino acid sequence set forth in SEQ ID NO:2;
(f) a nucleic acid molecule which encodes a polypeptide comprising an amino acid sequence at least about 90% identical to the amino acid sequence of SEQ ID NO:2;
(g) a nucleic acid molecule which encodes a fragment of a polypeptide comprising at least 250 contiguous amino acid residues of the amino acid sequence of SEQ ID NO:2; and
(h) a nucleic acid molecule comprising a nucleotide sequence which is complementary to the nucleotide sequence of the nucleic acid molecule of any one of subparts (a) to (g),
thereby identifying a compound capable of modulating nociception.

11. A method for identifying a compound capable of modulating nociception comprising:
(a) contacting a cell with a test compound; and
(b) assaying the ability of the test compound to modulate the activity of a polypeptide selected from the group consisting of:
(i) a fragment of a polypeptide comprising the amino acid sequence of SEQ ID NO:2, wherein the fragment comprises at least 250 contiguous amino acids of SEQ ID NO:2;
(ii) a polypeptide which is encoded by a nucleic acid molecule comprising a nucleotide sequence which is at least 99% identical to a nucleic acid comprising the nucleotide sequence of SEQ ID NO:1 or SEQ ID NO:3;
(iii) a polypeptide comprising an amino acid sequence which is at least 90% identical to the amino acid sequence of SEQ ID NO:2; and
(iv) a polypeptide comprising the amino acid sequence of SEQ ID NO:2, thereby identifying a compound capable of modulating nociception.

## Patentansprüche

1. Verfahren zum Identifizieren einer Verbindung, die an ein Polypeptid bindet oder die Fähigkeit eines Polypeptids moduliert, Kalzium über eine Membran zu transportieren, wobei das Polypeptid ausgewählt ist aus der Gruppe bestehend aus:
(i) einem Fragment eines Polypeptids umfassend die Aminosäuresequenz von SEQ ID NO:2, wobei das Fragment mindestens 250 zusammenhängende Aminosäuren von SEQ ID NO: 2 umfasst;
(ii) einem Polypeptid, das durch ein Nukleinsäuremolekül kodiert wird, das eine Nukleotidsequenz umfasst, die mindestens 99% identisch mit einer Nukleinsäure ist, die die Nukleotidsequenz von SEQ ID NO:1 oder SEQ ID NO:3 umfasst;
(iii) einem Polypeptid umfassend eine Aminosäuresequenz, die mindestens 90% identisch mit der Aminosäuresequenz von SEQ ID NO:2 ist; und
(iv) einem Polypeptid umfassend die Aminosäuresequenz von SEQ ID NO:2, umfassend:
(a) Kontaktieren des Polypeptids oder einer Zelle, die das Polypeptid expremiert, mit einer Testverbindung; und
(b) Bestimmen, ob die Verbindung an das Polypeptid bindet oder die Fähigkeit des Polypeptids moduliert, Kalzium über eine Membran zu transportieren, wodurch eine Verbindung identifiziert wird, die an das Polypeptid bindet oder die Fähigkeit des Polypeptids moduliert, Kalzium über eine Membran zu transportieren.

2. Verfahren zum Identifizieren eines Nukleinsäuremoleküls, das mit einer Schmerzstörung assoziiert ist, umfassend:
(a) Kontaktieren einer Probe umfassend Nukleinsäuremoleküle mit einer Hybridisierungssonde umfassend mindestens 25 zusammenhängende Nukleotide von SEQ ID NO:1; und
(b) Detektieren des Vorliegens eines Nukleinsäuremoleküls in der Probe, das an die Sonde hybridisiert, wodurch ein Nukleinsäuremolekül identifiziert wird, das mit einer Schmerzstörung assoziiert ist.

3. Verfahren zum Identifizieren eines Nukleinsäuremoleküls, das mit einer Schmerzstörung assoziiert ist, umfassend:
(a) Kontaktieren einer Probe umfassend Nukleinsäuremoleküle mit einem ersten und zweiten Amplifikationsprimer, wobei der erste Primer mindestens 25 zusammenhängende Nukleotide von SEQ ID NO:1 umfasst und der zweite Primer mindestens 25 zusammenhängende Nukleotide vom Komplement von SEQ ID NO:1 umfasst;
(b) Inkubieren der Probe unter Bedingungen, die Nukleinsäureamplifikation ermöglichen; und
(c) Detektieren des Vorliegens eines Nukleinsäuremoleküls in der Probe, das amplifiziert ist, wodurch ein Nukleinsäuremolekül identifiziert wird, das mit einer Schmerzstörung assoziiert ist.

4. Verfahren zum Identifizieren eines Polypeptids, das mit einer Schmerzstörung assoziiert ist, umfassend:
(a) Kontaktieren einer Probe umfassend Polypeptide mit einem Antikörper, der selektiv an ein Polypeptid von SEQ ID NO:2 bindet; und
(b) Detektieren des Vorliegens eines Polypeptids in der Probe, das an den Antikörper bindet, wodurch ein Polypeptid identifiziert wird, das mit einer Schmerzstörung assoziiert ist.

5. Verfahren von Anspruch 4, wobei der Antikörper detektierbar markiert ist.

6. Verfahren zum Identifizieren einer Verbindung, die in der Lage ist eine Schmerzstörung zu behandeln, umfassend das Testen der Fähigkeit der Verbindung die Expression einer Nukleinsäure zu modulieren, die ausgewählt ist aus der Gruppe bestehend aus:
(a) einem Nukleinsäuremolekül umfassend die Nukleotidsequenz, die in SEQ ID NO:1 dargestellt ist;
(b) einem Nukleinsäuremolekül umfassend die Nukleotidsequenz, die in SEQ ID NO:3 dargestellt ist;
(c) einem Nukleinsäuremolekül umfassend eine Nukleotidsequenz, die mindestens 99% identisch mit einer Nukleinsäure von SEQ ID NO:1 oder SEQ ID NO:3 ist.
(d) einem Nukleinsäuremolekül umfassend ein Fragment von mindestens 1850 Nukleotiden einer Nukleinsäure umfassend die Nukleotidsequenz von SEQ ID NO:1 oder SEQ ID NO:3;
(e) einem Nukleinsäuremolekül, das für ein Polypeptid kodiert, umfassend die Aminosäuresequenz, die in SEQ ID NO:2 dargestellt ist;
(f) einem Nukleinsäuremolekül, das für ein Polypeptid kodiert, umfassend eine Aminosäuresequenz, die mindestens 90% identisch mit der Aminosäuresequenz von SEQ ID NO:2 ist;
(g) einem Nukleinsäuremolekül, das für ein Fragment eines Polypeptids kodiert, umfassend mindestens 250 zusammenhängende Aminosäuren der Aminosäuresequenz von SEQ ID NO:2;
(h) einem Nukleinsäuremolekül umfassend eine Nukleinsäuresequenz, die komplementär ist zu der Nukleotidsequenz des Nukleinsäuremoleküls von jedem der Teilabschnitte (a) bis (g),
wodurch eine Verbindung identifiziert wird, die in der Lage ist eine Schmerzstörung zu behandeln.

7. Verfahren zum Identifizieren einer Verbindung, die in der Lage ist eine Schmerzstörung zu behandeln, umfassend das Testen der Fähigkeit der Verbindung die Kalziumkanalaktivität eines Polypeptids zu modulieren, das ausgewählt ist aus der Gruppe bestehend aus:
(a) einem Fragment eines Polypeptids umfassend die Aminosäuresequenz von SEQ ID NO:2, wobei das Fragment mindestens 250 zusammenhängende Aminosäuren der Aminosäuresequenz von SEQ ID NO:2 umfasst;
(b) einem Polypeptid, das durch ein Nukleinsäuremolekül kodiert wird, umfassend eine Nukleotidsequenz, die mindestens 99% identisch mit einer Nukleinsäure umfassend die Nukleotidsequenz von SEQ ID NO:1 oder SEQ ID NO:3 ist;
(c) einem Polypeptid, umfassend eine Aminosäuresequenz, die mindestens 90% identisch mit der Aminosäuresequenz von SEQ ID NO:2 ist; und
(d) einem Polypeptid, umfassend die Aminosäuresequenz von SEQ ID NO:2, wodurch eine Verbindung identifiziert wird, die in der Lage ist eine Schmerzstörung zu behandeln.

8. Verfahren von Anspruch 6, wobei die Verbindung die Expression von Nukleinsäuremolekül SEQ ID NO:1 oder 3 hemmt.

9. Verfahren von Anspruch 7, wobei die Verbindung die Aktivität eines Polypeptids SEQ ID NO:2 hemmt.

10. Verfahren zum Identifizieren einer Verbindung, die in der Lage ist Nozizeption zu behandeln, umfassend:
(a) Kontaktieren einer Zelle mit einer Testverbindung; und
(b) Testen der Fähigkeit der Verbindung die Expression einer Nukleinsäure zu modulieren, die ausgewählt ist aus der Gruppe bestehend aus:
(a) einem Nukleinsäuremolekül umfassend die Nukleotidsequenz, die in SEQ ID NO:1 dargestellt ist;
(b) einem Nukleinsäuremolekül umfassend die Nukleotidsequenz, die in SEQ ID NO:3 dargestellt ist;
(c) einem Nukleinsäuremolekül umfassend eine Nukleotidsequenz, die mindestens 99% identisch mit einer Nukleinsäure von SEQ ID NO:1 oder SEQ ID NO:3 ist.
(d) einem Nukleinsäuremolekül umfassend ein Fragment von mindestens 1850 Nukleotiden einer Nukleinsäure umfassend die Nukleotidsequenz von SEQ ID NO:1 oder SEQ ID NO:3;
(e) einem Nukleinsäuremolekül, das für ein Polypeptid kodiert, umfassend die Aminosäuresequenz, die in SEQ ID NO:2 dargestellt ist;
(f) einem Nukleinsäuremolekül, das für ein Polypeptid kodiert, umfassend eine Aminosäuresequenz, die mindestens 90% identisch mit der Aminosäuresequenz von SEQ ID NO:2 ist;
(g) einem Nukleinsäuremolekül, das für ein Fragment eines Polypeptids kodiert, umfassend mindestens 250 zusammenhängende Aminosäuren der Aminosäuresequenz von SEQ ID NO:2; und
(h) einem Nukleinsäuremolekül umfassend eine Nukleinsäuresequenz, die komplementär ist zu der Nukleotidsequenz des Nukleinsäuremoleküls von jedem der Teilabschnitte (a) bis (g), wodurch eine Verbindung identifiziert wird, die in der Lage ist Nozizeption zu behandeln.

11. Verfahren zum Identifizieren einer Verbindung, die in der Lage ist Nozizeption zu modulieren, umfassend:
(a) Kontaktieren einer Zelle mit einer Testverbindung; und
(b) das Testen der Fähigkeit der Verbindung die Aktivität eines Polypeptids zu modulieren, das ausgewählt ist aus der Gruppe bestehend aus:
(i) einem Fragment eines Polypeptids umfassend die Aminosäuresequenz von SEQ ID NO:2, wobei das Fragment mindestens 250 zusammenhängende Aminosäuren der Aminosäuresequenz von SEQ ID NO:2 umfasst;
(ii) einem Polypeptid, das durch ein Nukleinsäuremolekül kodiert wird, umfassend eine Nukleotidsequenz, die mindestens 99% identisch mit einer Nukleinsäure umfassend die Nukleotidsequenz von SEQ ID NO:1 oder SEQ ID NO:3 ist;
(iii) einem Polypeptid, umfassend eine Aminosäuresequenz, die mindestens 90% identisch mit der Aminosäuresequenz von SEQ ID NO:2 ist; und
(iv) einem Polypeptid, umfassend die Aminosäuresequenz von SEQ ID NO:2, wodurch eine Verbindung identifiziert wird, die in der Lage ist Nozizeption zu modulieren.

## Revendications

1. - Procédé d'identification d'un composé qui se lie à un polypeptide ou module l'aptitude d'un polypeptide à transporter le calcium à travers une membrane, dans lequel le polypeptide est choisi dans le groupe constitué par :
(i) un fragment d'un polypeptide comprenant la séquence d'acides aminés de SEQ ID NO : 2, le fragment comprenant au moins 250 acides aminés contigus de SEQ ID NO : 2 ;
(ii) un polypeptide qui est codé par une molécule d'acide nucléique comprenant une séquence nucléotidique qui est identique à au moins 99 % à un acide nucléique comprenant la séquence nucléotidique de SEQ ID NO : 1 ou SEQ ID NO : 3 ;
(iii) un polypeptide comprenant une séquence d'acides aminés qui est identique à au moins 90 % à la séquence d'acides aminés de SEQ ID NO : 2 ; et
(iv) un polypeptide comprenant la séquence d'acides aminés de SEQ ID NO : 2,
comprenant :
(a) la mise en contact du polypeptide ou d'une cellule exprimant le polypeptide avec un composé d'essai ; et
(b) la détermination de savoir si ou non le composé se lie au polypeptide ou module l'aptitude du polypeptide à transporter le calcium à travers une membrane, permettant ainsi d'identifier un composé qui se lie au polypeptide ou module l'aptitude du polypeptide à transporter le calcium à travers une membrane.

2. - Procédé d'identification d'une molécule d'acide nucléique associée à une affection douloureuse comprenant :
(a) la mise en contact d'un échantillon comprenant des molécules d'acide nucléique avec une sonde d'hybridation comprenant au moins 25 nucléotides contigus de SEQ ID NO : 1 ; et
(b) la détection de la présence d'une molécule d'acide nucléique dans ledit échantillon qui s'hybride à ladite sonde, permettant ainsi d'identifier une molécule d'acide nucléique associée avec une affection douloureuse.

3. - Procédé d'identification d'un acide nucléique associé à une affection douloureuse comprenant :
(a) la mise en contact d'un échantillon comprenant des molécules d'acide nucléique avec une première et une seconde amorce d'amplification, ladite première amorce comprenant au moins 25 nucléotides contigus de SEQ ID NO : 1 et ladite seconde amorce comprenant au moins 25 nucléotides contigus provenant du complément de SEQ ID NO : 1 ;
(b) l'incubation dudit échantillon dans des conditions qui permettent une amplification d'acide nucléique ; et
(c) la détection de la présence d'une molécule d'acide nucléique dans ledit échantillon qui est amplifiée, permettant ainsi d'identifier une molécule d'acide nucléique associée à une affection douloureuse.

4. - Procédé d'identification d'un polypeptide associé avec une affection douloureuse, comprenant :
(a) la mise en contact d'un échantillon comprenant des polypeptides avec un anticorps qui se lie de façon sélective à un polypeptide de SEQ ID NO : 2 ; et
(b) la détection de la présence d'un polypeptide dans ledit échantillon qui se lie audit anticorps, permettant ainsi d'identifier un polypeptide associé à une affection douloureuse.

5. - Procédé selon la revendication 4, dans lequel ledit anticorps est marqué de façon détectable.

6. - Procédé d'identification d'un composé capable de traiter une affection douloureuse comprenant l'essai de l'aptitude du composé à moduler l'expression d'une molécule d'acide nucléique choisie dans le groupe constitué par :
(a) une molécule d'acide nucléique comprenant la séquence nucléotidique exposée dans SEQ ID NO : 1 ;
(b) une molécule d'acide nucléique comprenant la séquence nucléotidique exposée dans SEQ ID NO : 3 ;
(c) une molécule d'acide nucléique comprenant une séquence nucléotidique qui est identique à au moins 99 % à la séquence nucléotidique de SEQ ID NO : 1 ou SEQ ID NO : 3 ;
(d) une molécule d'acide nucléique comprenant un fragment d'au moins 1850 nucléotides d'un acide nucléique comprenant la séquence nucléotidique de SEQ ID NO : 1 ou SEQ ID NO : 3 ;
(e) une molécule d'acide nucléique qui code pour un polypeptide comprenant la séquence d'acides aminés exposée dans SEQ ID NO : 2 ;
(f) une molécule d'acide nucléique qui code pour un polypeptide comprenant une séquence d'acides aminés identique à au moins environ 90 % à la séquence d'acides aminés de SEQ ID NO : 2 ;
(g) une molécule d'acide nucléique qui code pour un fragment d'un polypeptide comprenant au moins 250 restes d'acides aminés contigus de la séquence d'acides aminés de SEQ ID NO : 2 ; et
(h) une molécule d'acide nucléique comprenant une séquence nucléotidique qui est complémentaire de la séquence nucléotidique de la molécule d'acide nucléique de l'une quelconque des sous-parties (a) à (g), permettant ainsi d'identifier un composé capable de traiter une affection douloureuse.

7. - Procédé pour identifier un composé capable de traiter une affection douloureuse comprenant le dosage de l'aptitude du composé à moduler l'activité du canal calcique d'un polypeptide choisi dans le groupe constitué par :
(a) un fragment d'un polypeptide comprenant la séquence d'acides aminés de SEQ ID NO : 2, le fragment comprenant au moins 250 acides aminés contigus de SEQ ID NO : 2 ;
(b) un polypeptide qui est codé par une molécule d'acide nucléique comprenant une séquence nucléotidique qui est identique à au moins 99 % à un acide nucléique comprenant la séquence nucléotidique de SEQ ID NO : 1 ou SEQ ID NO : 3 ;
(c) un polypeptide comprenant une séquence d'acides aminés qui est identique à au moins 90 % à la séquence d'acides aminés de SEQ ID NO : 2 ; et
(d) un polypeptide comprenant la séquence d'acides aminés de SEQ ID NO : 2, permettant ainsi d'identifier un composé capable de traiter une affection douloureuse.

8. - Procédé selon la revendication 6, dans lequel ledit composé inhibe l'expression de la molécule d'acide aminé de SEQ ID NO : 1 ou 3.

9. - Procédé selon la revendication 7, dans lequel ledit composé inhibe l'activité d'un polypeptide de SEQ ID NO : 2.

10. - Procédé d'identification d'un composé capable de moduler la nociception comprenant :
(a) la mise en contact d'une cellule avec un composé d'essai ; et
(b) l'essai de l'aptitude du composé d'essai à moduler l'expression d'un acide nucléique choisi dans le groupe constitué par :
(a) une molécule d'acide nucléique comprenant la séquence nucléotidique exposée dans SEQ ID NO : 1 ;
(b) une molécule d'acide nucléique comprenant la séquence nucléotidique exposée dans SEQ ID NO : 3 ;
(c) une molécule d'acide nucléique comprenant une séquence nucléotidique qui est identique à au moins 99 % à la séquence nucléotidique de SEQ ID NO : 1 ou SEQ ID NO : 3 ;
(d) une molécule d'acide nucléique comprenant un fragment d'au moins 1850 nucléotides d'un acide nucléique comprenant la séquence nucléotidique de SEQ ID NO : 1 ou SEQ ID NO : 3 ;
(e) une molécule d'acide nucléique qui code pour un polypeptide comprenant la séquence d'acides aminés exposée dans SEQ ID NO : 2 ;
(f) une molécule d'acide nucléique qui code pour un polypeptide comprenant une séquence d'acides aminés identique à au moins environ 90 % à la séquence d'acides aminés de SEQ ID NO : 2 ;
(g) une molécule d'acide nucléique qui code pour un fragment d'un polypeptide comprenant au moins 250 résidus d'acides aminés contigus de la séquence d'acides aminés de SEQ ID NO : 2 ; et
(h) une molécule d'acide nucléique comprenant une séquence nucléotidique qui est complémentaire de la séquence nucléotidique de la molécule d'acide nucléique de l'une quelconque des sous-parties (a) à (g),
permettant ainsi d'identifier un composé capable de moduler la nociception.

11. - Procédé d'identification d'un composé capable de moduler la nociception comprenant :
(a) la mise en contact d'une cellule avec un composé d'essai ; et
(b) l'essai de l'aptitude du composé d'essai à moduler l'activité d'un polypeptide choisi dans le groupe constitué par :
(i) un fragment d'un polypeptide comprenant la séquence d'acides aminés de SEQ ID NO : 2, le fragment comprenant au moins 250 acides aminés contigus de SEQ ID NO : 2 ;
(ii) un polypeptide qui est codé par une molécule d'acide nucléique comprenant une séquence nucléotidique qui est identique à au moins 99 % à un acide nucléique comprenant la séquence nucléotidique de SEQ ID NO : 1 ou SEQ ID NO : 3 ;
(iii) un polypeptide comprenant une séquence d'acides aminés qui est identique à au moins 90 % à la séquence d'acides aminés de SEQ ID NO : 2 et
(iv) un polypeptide comprenant la séquence d'acides aminés de SEQ ID NO : 2, permettant ainsi d'identifier un composé capable de moduler la nociception.


### SEQUENCE LISTING

<110> Millennium Pharmaceuticals, Inc.
<120> 54420, A NOVEL HUMAN CALCIUM CHANNEL
<130> MNI-125CPPC
<140>
   <141>
<150> US 09/544,797
   <151> 2000-04-07
<160> 7
<170> PatentIn Ver. 2.0
<210> 1
   <211> 2095
   <212> DNA
   <213> Homo sapiens
<220>
   <221> CDS
   <222> (141)..(1880)
<400> 1
gtcgacccac gcgtccgcgg acgcgtgggc ggagggtttg aagccgcgcc gcgagggagc 60
   gaggtcgcag tgacagcggc gggcgatcgg acccaggctg ccccgccgta cccgcctgcg 120
   tcccgcgctc ccgccccagc atg aca gcc ccg gcg ggt ccg cgc ggc tca gag 173
   acc gag cgg ctt ctg acc ccc aac ccc ggg tat ggg acc cag gcg ggg   221
   cct tca ccg gcc cct ccg aca ccc cca gaa gag gaa gac ctt cgc cgt   269
   cgt ctc aaa tac ttt ttc atg agt ccc tgc gac aag ttt cga gcc aag   317
   ggc cgc aag ccc tgc aag ctg atg ctg caa gtg gtc aag atc ctg gtg   365
   gtc acg gtg cag ctc atc ctg ttt ggg ctc agt aat cag ctg gct gtg   413
   aca ttc cgg gaa gag aac acc atc gcc ttc cga cac ctc ttc ctg ctg   461
   ggc tac tcg gac gga gcg gat gac acc ttc gca gcc tac acg cgg gag   509
   cag ctg tac cag gcc atc ttc cat gct gtg gac cag tac ctg gcg ttg    557
   cct gac gtg tca ctg ggc cgg tat gcg tat gtc cgt ggt ggg ggt gac    605
   cct tgg acc aat ggc tca ggg ctt gct ctc tgc cag cgg tac tac cac    653
   cga ggc cac gtg gac ccg gcc aac gac aca ttt gac att gat ccg atg    701
   gtg gtt act gac tgc atc cag gtg gat ccc ccc gag cgg ccc cct ccg    749
   ccc ccc agc gac gat ctc acc ctc ttg gaa agc agc tcc agt tac aag    797
   aac ctc acg ctc aaa ttc cac aag ctg gtc aat gtc acc atc cac ttc    845
   cgg ctg aag acc att aac ctc cag agc ctc atc aat aat gag atc ccg    893
   gac tgc tat acc ttc agc gtc ctg atc acg ttt gac aac aaa gca cac    941
   agt ggg cgg atc ccc atc agc ctg gag acc cag gcc cac atc cag gag    989
   tgt aag cac ccc agt gtc ttc cag cac gga gac aac agc ttc cgg ctc    1037
   ctg ttt gac gtg gtg gtc atc ctc acc tgc tcc ctg tcc ttc ctc ctc    1085
   tgc gcc cgc tca ctc ctt cga ggc ttc ctg ctg cag aac gag ttt gtg    1133
   ggg ttc atg tgg cgg cag cgg gga cgg gtc atc agc ctg tgg gag cgg    1181
   ctg gaa ttt gtc aat ggc tgg tac atc ctg ctc gtc acc agc gat gtg    1229
   ctc acc atc tcg ggc acc atc atg aag atc ggc atc gag gcc aag aac    1277
   ttg gcg agc tac gac gtc tgc agc atc ctc ctg ggc acc tcg acg ctg    1325
   ctg gtg tgg gtg ggc gtg atc cgc tac ctg acc ttc ttc cac aac tac    1373
   aat atc ctc atc gcc aca ctg cgg gtg gcc ctg ccc agc gtc atg cgc    1421
   ttc tgc tgc tgc gtg gct gtc atc tac ctg ggc tac tgc ttc tgt ggc    1469
   tgg atc gtg ctg ggg ccc tat cat gtg aag ttc cgc tca ctc tcc atg    1517
   gtg tct gag tgc ctg ttc tcg ctc atc aat ggg gac gac atg ttt gtg    1565
   acg ttc gcc gcc atg cag gcg cag cag ggc cgc agc agc ctg gtg tgg    1613
   ctc ttc tcc cag ctc tac ctt tac tcc ttc atc agc ctc ttc atc tac    1661
   atg gtg ctc agc ctc ttc atc gcg ctc atc acc ggc gcc tac gac acc    1709
   atc aag cat ccc ggc ggc gca ggc gca gag gag agc gag ctg cag gcc    1757
   tac atc gca cag tgc cag gac agc ccc acc tcc ggc aag ttc cgc cgc    1805
   ggg agc ggc tcg gcc tgc agc ctt ctc tgc tgc tgc gga agg gac ccc    1853
   tcg gag gag cat tcg ctg ctg gtg aat tgattcgacc tgactgccgt          1900
   tggaccgtag gccctggact gcagagaccc ccgccccgac cccgcttatt tatttgtagg 1960
   gtttgctttt aaggatcggc tccctgtcgc gcccgaggag ggcctggacc tttcgtgtcg 2020
   gacccttggg ggcggggaga ctgggtggga aggtgtgaat aaaagggaaa ttaaaaaaaa 2080
   aaaaaaaatt ttaaa 2095
<210> 2
   <211> 580
   <212> PRT
   <213> Homo sapiens
<400> 2
<210> 3
   <211> 1740
   <212> DNA
   <213> Homo sapiens
<220>
   <221> CDS
   <222> (1)..(1740)
<400> 3
atg aca gcc ccg gcg ggt ccg cgc ggc tca gag acc gag cgg ctt ctg    48
   acc ccc aac ccc ggg tat ggg acc cag gcg ggg cct tca ccg gcc cct    96
   ccg aca ccc cca gaa gag gaa gac ctt cgc cgt cgt ctc aaa tac ttt   144
   ttc atg agt ccc tgc gac aag ttt cga gcc aag ggc cgc aag ccc tgc   192
   aag ctg atg ctg caa gtg gtc aag atc ctg gtg gtc acg gtg cag ctc   240
   atc ctg ttt ggg ctc agt aat cag ctg gct gtg aca ttc cgg gaa gag   288
   aac acc atc gcc ttc cga cac ctc ttc ctg ctg ggc tac tcg gac gga   336
   gcg gat gac acc ttc gca gcc tac acg cgg gag cag ctg tac cag gcc   384
   atc ttc cat gct gtg gac cag tac ctg gcg ttg cct gac gtg tca ctg   432
   ggc cgg tat gcg tat gtc cgt ggt ggg ggt gac cct tgg acc aat ggc   480
   tca ggg ctt gct ctc tgc cag cgg tac tac cac cga ggc cac gtg gac   528
   ccg gcc aac gac aca ttt gac att gat ccg atg gtg gtt act gac tgc   576
   atc cag gtg gat ccc ccc gag cgg ccc cct ccg ccc ccc agc gac gat   624
   ctc acc ctc ttg gaa agc agc tcc agt tac aag aac ctc acg ctc aaa      672
   ttc cac aag ctg gtc aat gtc acc atc cac ttc cgg ctg aag acc att      720
   aac ctc cag agc ctc atc aat aat gag atc ccg gac tgc tat acc ttc      768
   agc gtc ctg atc acg ttt gac aac aaa gca cac agt ggg cgg atc ccc      816
   atc agc ctg gag acc cag gcc cac atc cag gag tgt aag cac ccc agt      864
   gtc ttc cag cac gga gac aac agc ttc cgg ctc ctg ttt gac gtg gtg      912
   gtc atc ctc acc tgc tcc ctg tcc ttc ctc ctc tgc gcc cgc tca ctc      960
   ctt cga ggc ttc ctg ctg cag aac gag ttt gtg ggg ttc atg tgg cgg     1008
   cag cgg gga cgg gtc atc agc ctg tgg gag cgg ctg gaa ttt gtc aat     1056
   ggc tgg tac atc ctg ctc gtc acc agc gat gtg ctc acc atc tcg ggc     1104
   acc atc atg aag atc ggc atc gag gcc aag aac ttg gcg agc tac gac     1152
   gtc tgc agc atc ctc ctg ggc acc tcg acg ctg ctg gtg tgg gtg ggc     1200
   gtg atc cgc tac ctg acc ttc ttc cac aac tac aat atc ctc atc gcc     1248
   aca ctg cgg gtg gcc ctg ccc agc gtc atg cgc ttc tgc tgc tgc gtg     1296
   gct gtc atc tac ctg ggc tac tgc ttc tgt ggc tgg atc gtg ctg ggg     1344
   ccc tat cat gtg aag ttc cgc tca ctc tcc atg gtg tct gag tgc ctg     1392
   ttc tcg ctc atc aat ggg gac gac atg ttt gtg acg ttc gcc gcc atg     1440
   cag gcg cag cag ggc cgc agc agc ctg gtg tgg ctc ttc tcc cag ctc     1488
   tac ctt tac tcc ttc atc agc ctc ttc atc tac atg gtg ctc agc ctc     1536
   ttc atc gcg ctc atc acc ggc gcc tac gac acc atc aag cat ccc ggc     1584
   ggc gca ggc gca gag gag agc gag ctg cag gcc tac atc gca cag tgc     1632
   cag gac agc ccc acc tcc ggc aag ttc cgc cgc ggg agc ggc tcg gcc     1680
   tgc agc ctt ctc tgc tgc tgc gga agg gac ccc tcg gag gag cat tcg     1728
   ctg ctg gtg aat                                                     1740
<210> 4
   <211> 764
   <212> PRT
   <213> Homo sapiens
<400> 4
   Met Thr Ser Pro Ser Ser Ser Pro Val Phe Arg Leu Glu Thr Leu Asp
   1 5 10 15
   Gly Gly Gln Glu Asp Gly Ser Glu Ala Asp Arg Gly Lys Leu Asp Phe
   20 25 30
   Gly Ser Gly Leu Pro Pro Met Glu Ser Gln Phe Gln Gly Glu Asp Arg
   35 40 45
   Lys Phe Ala Pro Gln Ile Arg Val Asn Leu Asn Tyr Arg Lys Gly Thr
   50 55 60
   Gly Ala Ser Gln Pro Asp Pro Asn Arg Phe Asp Arg Asp Arg Leu Phe
   65 70 75 80
   Asn Ala Val Ser Arg Gly Val Pro Glu Asp Leu Ala Gly Leu Pro Glu
                       85                  90                  95
   Tyr Leu Ser Lys Thr Ser Lys Tyr Leu Thr Asp Ser Glu Tyr Thr Glu
               100                 105                 110
   Gly Ser Thr Gly Lys Thr Cys Leu Met Lys Ala Val Leu Asn Leu Lys
           115                 120                 125
   Asp Gly Val Asn Ala Cys Ile Leu Pro Leu Leu Gln Ile Asp Arg Asp
       130                 135                 140
   Ser Gly Asn Pro Gln Pro Leu Val Asn Ala Gln Cys Thr Asp Asp Tyr
   145                 150                 155                 160
   Tyr Arg Gly His Ser Ala Leu His Ile Ala Ile Glu Lys Arg Ser Leu
                   165                 170                 175
   Gln Cys Val Lys Leu Leu Val Glu Asn Gly Ala Asn Val His Ala Arg
               180                 185                 190
   Ala Cys Gly Arg Phe Phe Gln Lys Gly Gln Gly Thr Cys Phe Tyr Phe
           195                 200                 205
   Gly Glu Leu Pro Leu Ser Leu Ala Ala Cys Thr Lys Gln Trp Asp Val
       210                 215                 220
   Val Ser Tyr Leu Leu Glu Asn Pro His Gln Pro Ala Ser Leu Gln Ala
   225                 230                 235                 240
   Thr Asp Ser Gln Gly Asn Thr Val Leu His Ala Leu Val Met Ile Ser
                   245                 250                 255
   Asp Asn Ser Ala Glu Asn Ile Ala Leu Val Thr Ser Met Tyr Asp Gly
               260                 265                 270
   Leu Leu Gln Ala Gly Ala Arg Leu Cys Pro Thr Val Gln Leu Glu Asp
           275                 280                 285
   Ile Arg Asn Leu Gln Asp Leu Thr Pro Leu Lys Leu Ala Ala Lys Glu
       290                 295                 300
   Gly Lys Ile Glu Ile Phe Arg His Ile Leu Gln Arg Glu Phe Ser Gly
   305                 310                 315                 320
   Leu Ser His Leu Ser Arg Lys Phe Thr Glu Trp Cys Tyr Gly Pro Val
                   325                 330                 335
   Arg Val Ser Leu Tyr Asp Leu Ala Ser Val Asp Ser Cys Glu Glu Asn
               340                 345                 350
   Ser Val Leu Glu Ile Ile Ala Phe His Cys Lys Ser Pro His Arg His
           355                 360                 365
   Arg Met Val Val Leu Glu Pro Leu Asn Lys Leu Leu Gln Ala Lys Trp
       370                 375                 380
   Asp Leu Leu Ile Pro Lys Phe Phe Leu Asn Phe Leu Cys Asn Leu Ile
   385                 390                 395                 400
   Tyr Met Phe Ile Phe Thr Ala Val Ala Tyr His Gln Pro Thr Leu Lys
                   405                 410                 415
   Lys Gln Ala Ala Pro His Leu Lys Ala Glu Val Gly Asn Ser Met Leu
   420 425 430
   Leu Thr Gly His Ile Leu Ile Leu Leu Gly Gly Ile Tyr Leu Leu Val
   435 440 445
   Gly Gln Leu Trp Tyr Phe Trp Arg Arg His Val Phe Ile Trp Ile Ser
   450 455 460
   Phe Ile Asp Ser Tyr Phe Glu Ile Leu Phe Leu Phe Gln Ala Leu Leu
   465 470 475 480
   Thr Val Val Ser Gln Val Leu Cys Phe Leu Ala Ile Glu Trp Tyr Leu
   485 490 495
   Pro Leu Leu Val Ser Ala Leu Val Leu Gly Trp Leu Asn Leu Leu Tyr
   500 505 510
   Tyr Thr Arg Gly Phe Gln His Thr Gly Ile Tyr Ser Val Met Ile Gln
   515 520 525
   Lys Val Ile Leu Arg Asp Leu Leu Arg Phe Leu Leu Ile Tyr Leu Val
   530 535 540
   Phe Leu Phe Gly Phe Ala Val Ala Leu Val Ser Leu Ser Gln Glu Ala
   545 550 555 560
   Trp Arg Pro Glu Ala Pro Thr Gly Pro Asn Ala Thr Glu Ser Val Gln
   565 570 575
   Pro Met Glu Gly Gln Glu Asp Glu Gly Asn Gly Ala Gln Tyr Arg Gly
   580 585 590
   Ile Leu Glu Ala Ser Leu Glu Leu Phe Lys Phe Thr Ile Gly Met Gly
   595 600 605
   Glu Leu Ala Phe Gln Glu Gln Leu His Phe Arg Gly Met Val Leu Leu
   610 615 620
   Leu Leu Leu Ala Tyr Val Leu Leu Thr Tyr Ile Leu Leu Leu Asn Met
   625 630 635 640
   Leu Ile Ala Leu Met Ser Glu Thr Val Asn Ser Val Ala Thr Asp Ser
   645 650 655
   Trp Ser Ile Trp Lys Leu Gln Lys Ala Ile Ser Val Leu Glu Met Glu
   660 665 670
   Asn Gly Tyr Trp Trp Cys Arg Lys Lys Gln Arg Ala Gly Val Met Leu
   675 680 685
   Thr Val Gly Thr Lys Pro Asp Gly Ser Pro Asp Glu Arg Trp Cys Phe
   690 695 700
   Arg Val Glu Glu Val Asn Trp Ala Ser Trp Glu Gln Thr Leu Pro Thr
   705 710 715 720
   Leu Cys Glu Asp Pro Ser Gly Ala Gly Val Pro Arg Thr Leu Glu Asn
   725 730 735
   Pro Val Leu Ala Ser Pro Pro Lys Glu Asp Glu Asp Gly Ala Ser Glu
   740 745 750
   Glu Asn Tyr Val Pro Val Gln Leu Leu Gln Ser Asn
   755 760
<210> 5
   <211> 764
   <212> PRT
   <213> Homo sapiens
<220>
<223> Xaa at position 667 may be any amino acid
<400> 5
   Met Thr Ser Pro Ser Ser Ser Pro Val Phe Arg Leu Glu Thr Leu Asp
   1 5 10 15
   Gly Gly Gln Glu Asp Gly Ser Glu Ala Asp Arg Gly Lys Leu Asp Phe
   20 25 30
   Gly Ser Gly Leu Pro Pro Met Glu Ser Gln Phe Gln Gly Glu Asp Arg
   35 40 45
   Lys Phe Ala Ser Gln Ile Arg Val Asn Leu Asn Tyr Arg Lys Gly Thr
   50 55 60
   Gly Ala Ser Gln Pro Asp Pro Asn Arg Phe Asp Arg Asp Arg Leu Phe
   65 70 75 80
   Asn Val Val Ser Arg Gly Val Pro Glu Asp Leu Ala Gly Leu Pro Glu
   85 90 95
   Tyr Leu Ser Lys Thr Ser Lys Tyr Leu Thr Asp Ser Glu Tyr Thr Glu
   100 105 110
   Gly Ser Thr Gly Lys Thr Cys Leu Met Lys Ala Val Leu Asn Leu Lys
   115 120 125
   Asp Gly Val Asn Ala Cys Ile Leu Pro Leu Leu Gln Ile Asp Arg Asp
   130 135 140
   Ser Gly Asn Pro Gln Pro Leu Val Asn Ala Gln Cys Thr Asp Asp Tyr
   145 150 155 160
   Tyr Arg Gly His Ser Ala Leu His Ile Ala Ile Glu Lys Arg Ser Leu
   165 170 175
   Gln Cys Val Lys Leu Leu Val Glu Asn Gly Ala Asn Val His Ala Arg
   180 185 190
   Ala Cys Gly Arg Phe Phe Gln Asn Gly Gln Gly Thr Cys Phe Tyr Phe
   195 200 205
   Gly Glu Leu Pro Leu Ser Leu Ala Ala Cys Thr Lys Gln Trp Asp Val
   210 215 220
   Val Ser Tyr Leu Leu Glu Asn Pro His Gln Pro Ala Ser Leu Gln Ala
   225 230 235 240
   Thr Asp Ser Gln Gly Asn Thr Val Leu His Ala Leu Val Met Ile Ser
                   245                 250                 255
   Asp Asn Ser Ala Glu Asn Ile Ala Leu Val Thr Ser Met Tyr Asp Gly
               260                 265                 270
   Leu Leu Gln Ala Gly Ala Arg Leu Cys Pro Thr Val Gln Leu Glu Asp
           275                 280                 285
   Ile Arg Asn Leu Gln Asp Leu Thr Pro Leu Lys Leu Ala Ala Lys Glu
       290                 295                 300
   Gly Lys Ile Glu Ile Phe Arg His Ile Leu Gln Arg Glu Phe Ser Gly
   305                 310                 315                 320
   Leu Ser His Leu Ser Arg Lys Phe Thr Glu Trp Cys Tyr Gly Pro Val
                   325                 330                 335
   Arg Val Ser Leu Tyr Asp Leu Ala Ser Val Asp Ser Cys Glu Glu Asn
               340                 345                 350
   Ser Val Leu Glu Ile Ile Ala Phe His Cys Lys Ser Pro His Arg His
           355                 360                 365
   Arg Met Val Val Leu Glu Pro Leu Asn Lys Leu Leu Gln Ala Lys Trp
       370                 375                 380
   Asp Leu Leu Ile Pro Lys Phe Phe Leu Asn Phe Leu Cys Asn Leu Ile
   385                 390                 395                 400
   Tyr Met Phe Ile Phe Thr Ala Val Ala Tyr His Gln Pro Thr Leu Lys
                   405                 410                 415
   Lys Gln Ala Ala Pro His Leu Lys Ala Glu Val Gly Asn Ser Met Leu
               420                 425                 430
   Leu Thr Gly His Ile Leu Ile Leu Leu Gly Gly Ile Tyr Leu Leu Val
           435                 440                 445
   Gly Gln Leu Trp Tyr Phe Trp Arg Arg His Leu Phe Ile Trp Ile Ser
       450                 455                 460
   Tyr Thr Asp Ser Tyr Phe Glu Ile Leu Phe Leu Phe His Ser Leu Leu
   465                 470                 475                 480
   Thr Val Val Ser Leu Val Leu Cys Phe Leu Val Ile Glu Trp Tyr Leu
                   485                 490                 495
   Pro Leu Leu Val Ser Ala Leu Val Leu Gly Trp Leu Asn Leu Leu Tyr
               500                 505                 510
   Tyr Thr Arg Gly Phe Gln His Thr Gly Ile Tyr Ser Val Met Ile Gln
           515                 520                 525
   Lys Val Ile Leu Arg Asp Met Val Arg Phe Leu Val Ile Tyr Leu Val
       530                 535                 540
   Phe Leu Phe Gly Phe Ala Val Ala Leu Val Ser Leu Ser Gln Glu Ala
   545                 550                 555                 560
   Trp Arg Pro Glu Ala Pro Thr Gly Pro Asn Ala Thr Glu Ser Val Gln
                           565                     570                     575
   Pro Met Glu Gly Gln Glu Asp Glu Gly Asn Gly Ala Gln Tyr Arg Gly
                   580                     585                     590
   Ile Leu Glu Ala Ser Leu Glu Leu Phe Lys Phe Thr Ile Gly Met Gly
               595                     600                     605
   Glu Leu Ala Phe Gln Glu Gln Leu His Phe Arg Gly Met Val Leu Leu
       610                     615                     620
   Leu Leu Leu Ala Tyr Val Leu Leu Thr Tyr Ile Leu Leu Leu Asn Met
   625                     630                     635                     640
   Leu Ile Ala Leu Met Ser Glu Thr Val Asn Ser Val Ala Thr Asp Ser
                   645                     650                     655
   Trp Ser Ile Trp Lys Leu Gln Lys Ala Ile Xaa Val Leu Glu Met Glu
               660                     665                     670
   Asn Gly Tyr Trp Trp Cys Arg Lys Lys Gln Arg Ala Gly Val Met Leu
           675                     680                     685
   Thr Val Gly Thr Lys Pro Asp Gly Ser Pro Asp Glu Arg Trp Cys Phe
       690                     695                     700
   Arg Val Glu Glu Val Asn Trp Ala Ser Trp Glu Gln Thr Leu Pro Thr
   705                     710                     715                     720
   Leu Cys Glu Asp Pro Ser Gly Ala Gly Val Pro Arg Thr Leu Glu Asn
                   725                     730                     735
   Pro Val Leu Ala Ser Pro Pro Lys Glu Asp Glu Asp Gly Ala Ser Glu
               740                     745                     750
   Glu Asn Tyr Val Pro Val Gln Leu Leu Gln Ser Asn
           755                     760
<210> 6
   <211> 966
   <212> PRT
   <213> Homo sapiens
<400> 6
   Met Val Asn Ser Arg Arg Val Gln Pro Gln Pro Pro Gly Asp Ala Gly
   1 5 10 15
   Arg Ser Pro Ala Pro Arg Ala Ser Gly Pro Gly Arg Leu Val Ala Gly
   20 25 30
   Gly Ala Gly Leu Ala Val Pro Gly Gly Leu Gly Glu Gln Arg Gly Leu
   35 40 45
   Glu Ile Glu Met Glu Arg Ile Arg Gln Ala Ala Ala Arg Asp Pro Pro
   50 55 60
   Ala Gly Ala Ser Ala Ser Pro Ser Pro Pro Leu Ser Ser Cys Ser Arg
   65 70 75 80
   Gln Ala Trp Ser Arg Asp Asn Pro Gly Phe Glu Ala Glu Glu Asp Asp
                       85                  90                  95
   Asp Asp Asp Glu Val Glu Gly Glu Glu Gly Gly Met Val Val Glu Met
               100                 105                 110
   Asp Val Glu Trp Arg Pro Gly Ser Arg Arg Ser Ala Ser Ser Ser Ala
           115                 120                 125
   Val Ser Ser Val Gly Ala Arg Gly Arg Gly Leu Gly Ser Tyr Arg Gly
       130                 135                 140
   Ala Ala His Leu Ser Gly Arg Arg Arg Arg Leu Glu Asp Gln Gly Ala
   145                 150                 155                 160
   Gln Cys Pro Ser Pro Ala Gly Gly Gly Asp Pro Leu His Arg His Leu
                   165                 170                 175
   Pro Leu Glu Gly Gln Pro Pro Arg Val Ala Trp Ala Glu Arg Leu Val
               180                 185                 190
   Arg Gly Leu Arg Gly Leu Trp Gly Thr Arg Leu Met Glu Glu Ser Asn
           195                 200                 205
   Ala Asn Arg Glu Lys Tyr Leu Lys Ser Val Leu Arg Glu Leu Val Thr
       210                 215                 220
   Tyr Leu Phe Phe Leu Val Val Leu Cys Ile Leu Thr Tyr Gly Met Met
   225                 230                 235                 240
   Ser Ser Asn Val Tyr Tyr Tyr Thr Arg Thr Leu Ser Gln Leu Phe Ile
                   245                 250                 255
   Asp Thr Pro Val Ser Lys Thr Glu Lys Thr Asn Phe Lys Thr Leu Ser
               260                 265                 270
   Ser Met Glu Asp Phe Trp Lys Phe Thr Glu Gly Ser Phe Leu Asp Gly
           275                 280                 285
   Leu Tyr Trp Lys Ala Gln Thr Ser Asn His Thr Gln Ala Asp Asn Arg
       290                 295                 300
   Ser Phe Ile Phe Tyr Glu Asn Leu Leu Leu Gly Val Pro Arg Leu Arg
   305                 310                 315                 320
   Gln Leu Arg Val Arg Asn Gly Ser Cys Ser Ile Pro Gln Asp Leu Arg
                   325                 330                 335
   Asp Glu Ile Lys Glu Cys Tyr Asp Val Tyr Ser Val Ser Ser Glu Asp
               340                 345                 350
   Arg Ala Pro Phe Gly Pro Arg Asn Gly Thr Ala Trp Met Tyr Thr Ser
           355                 360                 365
   Glu Lys Glu Leu Asn Gly Ser Ser His Trp Gly Ile Ile Ala Ser Tyr
       370                 375                 380
   Ser Gly Ala Gly Tyr Tyr Leu Asp Leu Ser Arg Thr Arg Glu Glu Thr
   385                 390                 395                 400
   Ala Ala Gln Leu Ala Gly Leu Arg Arg Asn Phe Trp Leu Asp Arg Gly
                   405                 410                 415
   Thr Arg Ala Ala Phe Ile Asp Phe Ser Val Tyr Asn Ala Asn Ile Asn
                   420                 425                 430
   Leu Phe Cys Val Val Arg Leu Leu Ala Glu Phe Pro Ala Thr Gly Gly
           435                 440                 445
   Val Val Pro Ser Trp Gln Phe Gln Pro Val Lys Leu Ile Arg Tyr Val
       450                 455                 460
   Thr Ala Phe Asp Phe Phe Leu Ala Ala Cys Glu Ile Ile Phe Cys Phe
   465                 470                 475                 480
   Phe Ile Ile Tyr Tyr Val Val Glu Glu Ile Leu Glu Ile Arg Ile His
                   485                 490                 495
   Arg Leu Ser Tyr Phe Arg Ser Phe Trp Asn Cys Leu Asp Val Val Ile
               500                 505                 510
   Val Val Leu Ser Val Val Ala Met Val Ile Asn Ile Tyr Arg Met Ser
           515                 520                 525
   Asn Ala Glu Gly Leu Leu Gln Phe Leu Glu Asp Gln Asn Ser Phe Pro
       530                 535                 540
   Asn Phe Glu His Val Ala Tyr Trp Gln Ile Gln Phe Asn Asn Ile Ser
   545                 550                 555                 560
   Ala Val Met Val Phe Leu Val Trp Ile Lys Leu Phe Lys Phe Ile Asn
                   565                 570                 575
   Phe Asn Arg Thr Met Ser Gln Leu Ser Thr Thr Met Ser Arg Cys Ala
               580                 585                 590
   Lys Asp Leu Phe Gly Phe Thr Ile Met Phe Ser Ile Ile Phe Leu Ala
           595                 600                 605
   Tyr Ala Gln Leu Ala Tyr Leu Val Phe Gly Thr Gln Val Asp Asp Phe
       610                 615                 620
   Ser Thr Phe Gln Glu Cys Ile Phe Thr Gln Phe Arg Ile Ile Leu Gly
   625                 630                 635                 640
   Asp Ile Asn Phe Ala Glu Ile Glu Glu Ala Asn Arg Val Leu Gly Pro
                   645                 650                 655
   Leu Tyr Phe Thr Thr Phe Val Phe Phe Met Phe Phe Ile Leu Leu Asn
               660                 665                 670
   Met Phe Leu Ala Ile Ile Asn Asp Ser Tyr Ser Glu Val Lys Ser Ser
           675                 680                 685
   Val Ala Gln Gln Lys Ala Glu Met Glu Leu Ser Asp Leu Ile Arg Lys
       690                 695                 700
   Gly Cys Gln Lys Ala Leu Val Lys Leu Lys Leu Lys Arg Asn Thr Val
   705                 710                 715                 720
   Asp Ala Ile Ser Glu Ser Leu Arg Gln Gly Gly Gly Lys Leu Asn Phe
                   725                 730                 735
   Asp Glu Leu Arg Gln Asp Leu Lys Gly Glu Gly His Thr Asp Ala Glu
   740 745 750
   Ile Glu Ala Ile Phe Thr Lys Tyr Asp Gln Asp Gly Asp Gln Glu Leu
   755 760 765
   Thr Glu Arg Glu His Gln Gln Met Arg Asp Asp Leu Glu Lys Glu Arg
   770 775 780
   Glu Asp Leu Asp Leu Glu His Ser Ser Leu Pro Arg Pro Met Ser Ser
   785 790 795 800
   Arg Ser Phe Pro Arg Ser Leu Asp Asp Ser Glu Glu Glu Asp Asp Glu
   805 810 815
   Asp Ser Gly His Ser Ser Arg Arg Arg Gly Ser Ile Ser Ser Gly Val
   820 825 830
   Ser Tyr Glu Glu Phe Gln Val Leu Val Arg Arg Val Asp Arg Met Glu
   835 840 845
   His Ser Ile Gly Ser Ile Val Ser Lys Ile Asp Ala Val Ile Val Lys
   850 855 860
   Leu Glu Ile Met Glu Arg Ala Lys Leu Lys Arg Arg Glu Val Leu Gly
   865 870 875 880
   Arg Leu Leu Asp Gly Val Ala Glu Asp Ala Arg Leu Gly Arg Asp Ser
   885 890 895
   Glu Ile His Arg Glu Gln Met Glu Arg Leu Val Arg Glu Glu Leu Glu
   900 905 910
   Arg Trp Glu Ser Asp Asp Ala Ala Ser Gln Thr Gly His Gly Val Ser
   915 920 925
   Thr Gln Val Gly Leu Gly Gly Gln Pro His Pro Arg Asn Pro Arg Pro
   930 935 940
   Pro Ser Ser Gln Ser Ala Glu Gly Leu Glu Gly Gly Gly Gly Asn Gly
   945 950 955 960
   Ser Ala Asn Val His Ala
   965
<210> 7
   <211> 1533
   <212> PRT
   <213> Homo sapiens
<400> 7